# EUROPEAN PATENT SPECIFICATION

(11) **EP 1 497 465 B1**
(45) Date of publication and mention of the grant of the patent: **18.02.2009**
(21) Application number: 03728567.3
(22) Date of filing: 25.04.2003
(51) Int. Cl.: C12Q 1/68

(54) **CONSTANT LENGTH SIGNATURES FOR PARALLEL SEQUENCING OF POLYNUCLEOTIDES**
SIGNATUREN KONSTANTER LÄNGE FÜR DAS PARALLELE SEQUENZIEREN VON POLYNUKLEOTIDEN
SIGNATURES DE LONGUEUR CONSTANTE POUR LE SEQUENCAGE EN PARALLELE DE POLYNUCLEOTIDES

(30) Priority: 26.04.2002 US 375782 P
(43) Date of publication of application: 19.01.2005
(73) Proprietor: Solexa, Inc., Hayward, CA 94545 (US)
(72) Inventor: FISCHER, Achim, 69120 Heidelberg (DE); HIEMISCH, Holger, 69118 Heidelberg (DE); WILLIAMS, Steven, San Francisco, CA 94114 (US); BRENNER, Sydney, La Jolla, CA 92037 (US); WALKER, Roger, Benicia, CA 94510 (US); VERMAAS, Eric, San Leandro, CA 94577 (US); FU, Rongdian, El Cerrito, CA 94530 (US)
(74) Representative: White, Nina Louise
(86) International application number: PCT/US2003/013076
(87) International publication number: WO 2003/091416

(56) References cited:
- US-A- 5 629 179
- US-A- 6 013 445
- US-B1- 6 258 539
- US-B1- 6 274 320
- US-B1- 6 291 181
- BRENNER SYDNEY ET AL: "Gene expression analysis by massively parallel signature sequencing (MPSS) on microbead arrays" NATURE BIOTECHNOLOGY, NATURE PUBLISHING, US, vol. 18, no. 6, June 2000 (2000-06), pages 630-634, XP002215573 ISSN: 1087-0156

## Description

### Field of the Invention

The invention relates generally to methods for sorting and sequencing polynucleotides, and more particularly, to a method for sequencing many polynucleotides simultaneously, *e.g.* for comparison of source DNA populations.

### Background of the Invention

Parallel sequencing of large populations of polynucleotides is useful in such areas as genomic mapping, genetic identification, medical diagnostics, and the like. Such sequencing is facilitated by the provision of solid-phase supported libraries of polynucleotide fragments, wherein each fragment is attached to a separate microparticle in a clonal subpopulation, as disclosed, for example, in Brenner, U.S. Pat. No. 5,604,097, Brenner et al., PCT Pubn. No. WO 96/41011, and Albrecht et al., U.S. Patent No. 6,265,163. Such libraries, for use in the analysis of gene expression in a plurality of cells or tissues, can be constructed from cDNA libraries generated from cells or tissues of interest. For analysis of genetic variations between genomic DNA samples of individuals or populations of individuals, the libraries are derived from genomic DNA extracted from each of the individuals.

As explained more fully below, clonal subpopulations of sequences making up such libraries can be formed by a "solid phase cloning" procedure, disclosed in detail in Brenner, U.S. Pat. Nos. 5,604,097 and 5,763,175 and Brenner et al., PCT Pubn. No. WO 96/41 O11. Briefly, polynucleotide fragments are inserted into a library of tag vectors, which carry a repertoire of oligonucleotide tags, to form a vector library of tag-signature sequence conjugates. The vectors containing the tag-signature conjugates are amplified. The tags of the tag-DNA conjugates are then rendered single stranded, and the tag-signature conjugates are hybridized and ligated to tag complements attached to microparticles.

The loaded sequences can then be identified, preferably by a stepwise method which sequentially identifies nucleotides, or short sequences of nucleotides, along the length of the support-bound sequence. For identification, only a portion of the DNAs need be sequenced. In many cases, the portion may be as small as nine or ten nucleotides; see *e.g.* Velculescu et al. Science 270: 484-486 (1995). Such sequencing methods include those described, for example, in Brenner, U.S. Patent Nos. 5,604,097, 5,962,228, and 5,599,675. In one embodiment, the sequences are identified simultaneously in parallel by MPSS (massively parallel signature sequencing), as described below and in Brenner, U.S. Pat. No. 6,013,445 and Albrecht et al., PCT Pubn. No. WO 97/46704.

The above-described methods have been used for signature sequencing of nucleic acid populations, as described, for example, in U.S. Patent No. 6,013,445. The solid-phase sorted libraries of sequences can be sequenced in parallel to provide a signature or "fingerprint" of the nucleic acid population from which the library was derived. The libraries can also be used as reference libraries for differential analysis of multiple libraries by competitive hybridization, as described in U.S. Patent No. 6,265,163.

In previously reported methods of preparing the solid phase cloned libraries, the source DNA is first cleaved with a restriction endonuclease to produce fragments, which are then cloned into a tag-vector library, as described above. These fragments can vary considerably in length, leading to possible biases in processing, particularly in PCR amplification step(s) and, to some extent, in the loading and ligating of the tag-DNA, conjugates onto the complement-containing microparticles. Specifically, PCR amplification of nucleic acid fragments from a typical restriction digest is expected to introduce bias against longer species, as it is known that short DNA molecules are amplified preferentially in PCR.

The present invention provides a method of producing tag-DNA constructs or libraries in which the DNA signature components are all of the same length, thus eliminating the above noted bias during amplification of the sequences.

### Summary of the Invention

The invention provides, in one aspect, a method of preparing a library of same-length signature sequences from a source polynucleotide population. The method comprises the following steps, which will be described in more detail in succeeding sections:
(a) attaching to an end of each of a population of polynucleotides a first adapter (referred to herein as a "Q adapter") containing a recognition site for a first restriction endonuclease, such that the cleavage site for the endonuclease is within the polynucleotide,
   wherein the first restriction endonuclease is a type IIs restriction endonuclease having a cleavage site at least 16 nucleotides from its recognition site, and wherein the end to which the adaptor is attached is the same for each polynucleotide in the population, and is selected from: (i) the 5' end of a full length cDNA transcript, (ii) the 3' end of a cDNA transcript from which the polyA/polyT tract has been removed, (iii) the 5' end of a cDNA fragment produced by cleavage of cDNA with a restriction endonuclease; and (iv) the 3' end of a cDNA fragment produced by cleavage of cDNA with a restriction endonuclease;
(b) cleaving the polynucleotides With the first restriction endonuclease, to produce a population of adapter-signature conjugates, each containing a same-length signature sequence of source nucleic acid, at least six basepairs in length, having a newly cleaved end; and
(c) ligating to the newly cleaved ends of said signatures in said adapter-signature conjugates, a second adapter, said second adapter containing one or more of (i) a primer binding site and (ii) a recognition and cleavage site for a second restriction endonuclease, to produce a library of adapter-signature adapter constructs.
The constructs may then be digested with the second endonuclease and a restriction endonuclease effective to cleave the first adapter, to produce a library of same-length signature fragments flanked by cloning sites.

In one embodiment, the first restriction endonuclease is a type IIs restriction endonuclease, as discussed further below.

In selected embodiments, the end to which the first adaptor is attached is selected from: (i) the 5' end of a full length cDNA and (ii) the 3' end of a full length cDNA from which the polyA tract has been removed. In other embodiments, the end to which the first adaptor is attached is selected from: (iii) the 5' end of a cDNA augment produced by cleavage of cDNA with a restriction endonuclease, and (iv) the 3' end of a DNA fragment produced by cleavage of cDNA with a restriction endonuclease. In the latter case, a portion of the cDNA fragments of (iii) are preferably derived from the 3' regions of said source nucleic acid population. Similarly, a portion of the cDNA fragments of (iv) are preferably derived from the 5' regions of said source nucleic acid population. These fragments, representing the 3' regions or the 5' regions of the source nucleic acid population, respectively, are preferably isolated from other cDNA fragments following attachment of the first adapter.

In selected embodiments, the Q adapter is attached in solution phase in step (a) above; in other embodiments, one or more components of the reaction is bound to a solid phase support.

In a preferred embodiment, at least one adapter, and preferably each adapter, includes a binding site for a primer or polymerase, and the method further comprises, following step (c) and preceding step (d): removing the bottom strand of each adapter-signature construct; and regenerating the bottom strand, by reverse transcription, primer extension, or PCR amplification, preferably by PCR amplification.

Generally, the cleaved end of step a) has a single stranded overhang. Alternatively, the overhang is removed from the fragments prior to ligating the first adapter, in which case the first adapter has a blunt end.

The second adapter typically comprises a set of adapters containing single stranded overhangs of every possible sequence effective to hybridize with the single stranded overhang of the newly cleaved ends generated in step (b).

The first restriction endonuclease is a type IIs restriction endonuclease having a cleavage site at least 16 nucleotides from its recognition site. Examples include BpmI, MmeI, GsuI, and isoschizomers thereof.

The second restriction endonuclease is preferably a type IIs endonuclease having a four-base recognition site which produces an overhang of at least 2 nucleotides upon cleavage; more preferably, it produces a 5'-extension of at least 3 nucleotides upon cleavage. Examples include SfaNI, BspMI, BbvI, FokI, BsmFI, BbsI, and isoschizomers thereof.

The signature sequences can be sequenced in parallel by a process which includes:
attaching an oligonucleotide tag to each signature fragment, such that substantially all different signature fragments have different oligonucleotide tags attached, to form tag-signature conjugates;
contacting the tag-signature conjugates with a library of tag complements, each on a separate solid phase support, and hybridizing the tags to their respective complements, to form solid-phase supported clonal subpopulations of signature sequences; and
sequencing a plurality of the solid-phase supported signature sequences.
In a preferred procedure, the signature-containing inserts are ligated into a library of oligonucleotide tag-vectors, wherein each tag-vector comprises: a left restriction cleavage site, an oligonucleotide tag, a cloning site for insertion of the signature fragment, and a right restriction cleavage site; thereby forming a vector library of tag-signature conjugates, which is then replicated in a host organism.

Preferably, the number of different oligonucleotide tags in the tag-vector library is greater than the number of different fragments by a factor of at least 100, and the process further includes the step of taking a sample from the vector library, such that substantially all different polynucleotide fragments within the sample have different tags attached.

Further processing of the amplified tag-signature constructs may be carried out by loading the constructs onto solid phase supports, as follows: amplifying a sample of the constructs by PCR, preferably using a fluorescently labeled primer and a biotinylated primer, and purifying the amplicon by streptavidin capture; cleaving the tag-signature conjugates from the vector; removing the bottom strand of the tag component of the tag-signature conjugates; contacting the tag-signature conjugates with a library of tag complements, each on a separate solid phase support, thereby hybridizing the single stranded tags to their respective complements; and ligating the bottom strands of the signature fragments to the tag complements; thereby forming a library comprising solid-phase supported clonal subpopulations of each same-length signature sequence from the source polynucleotide population.

The fluorescent label (incorporated during PCR, above) can be used to sort loaded from unloaded solid phase supports by FACS (fluorescence activated cell sorting), then removed prior to sequencing.

In an alternative loading process, the PCR amplification step is omitted, the tag-signature constructs are cleaved and isolated (*e.g.* by electrophoretic separation) from the vector backbone, and a fluorescent adapter is ligated to the tag-signature conjugates, followed by "stripping" of the tag, hybridization and ligation to solid supports as above.

Still another method of such loading can be carried out as follows:
i) linearizing the vectors containing the tag-signature conjugates;
ii) replicating the top strands by *in vitro* transcription, reverse transcribing the bottom strands using a first biotin-labeled primer, and conducting second strand synthesis of the top strands using a second biotin-labeled primer;
iii) cleaving the vectors at the left restriction cleavage site, thereby removing the first biotin labels;
iv) binding the second biotin labels to a streptavidin support, and eluting the top strands from the support;
v) annealing a primer to a region of each top strand which is 3' of the signature;
vi) contacting the top strands of the tag-signature constructs with a library of tag complements, each on a separate solid phase support, thereby hybridizing the tags to their respective complements;
vii) replicating the signature portions of said top strands to form double stranded signatures, and
viii) ligating a strand containing the signature to the tag complement;
thereby forming a library comprising solid-phase supported clonal subpopulations of each same-length signature sequence from the source polynucleotide population.

In one embodiment of this latter procedure, the above-noted region 3' of the signature (step v) includes a restriction enzyme cleavage site at its 5' end, and the corresponding restriction enzyme is used to cleave the region from the signature, following the ligation of step viii.

With reference to the oligonucleotide tags, each tag is preferably selected from the same minimally cross-hybridizing set of oligonucleotides; in one embodiment, each tag consists of a plurality of subunits, each subunit consisting of an oligonucleotide of 3 to 9 nucleotides in length, where each subunit is selected from the same minimally cross-hybridizing set of oligonucleotides. Preferably, each of these subunits differs from every other subunit of the same minimally cross-hybridizing set by at least three nucleotides.

The disclosed methods of preparing same-length signatures and preparing solid phase libraries of such signatures can be used for comparison of one or more source polynucleotide populations, by sequencing a plurality of such solid-phase supported signature sequences, for each source polynucleotide population. In one embodiment, the sequencing is carried out by massively parallel signature sequencing (MPSS), as described further below.

Such sequencing may be directed to analysis of differentially regulated or expressed genes, where the source populations are cDNA libraries derived from expressed genes of each of a plurality of sources selected from different cells, tissues, or individuals. It may also be directed to analysis of genetic variations among individuals or populations of individuals, where the source populations are genomic DNA libraries derived from different individuals or populations of individuals. In the latter case, the genetic variations preferably occur at known or predictable locations or regions in the genomic DNA.

In a related embodiment, the disclosure provides kits for use in parallel sequencing of signature sequences from a sample nucleic acid population. Components of the kits comprise: (i) an oligonucleotide tag vector library, each tag vector in the library comprising: a left primer binding site, a left restriction cleavage site, an oligonucleotide tag selected from a minimally cross-hybridizing set of oligonucleotides, a cloning site for insertion of a signature fragment, a right restriction cleavage site, and a right primer binding site; (ii) a corresponding set of oligonucleotide tag complements, each bound to a spatially distinct solid phase support; and (iii) a population of signature inserts flanked bye cloning sites, each containing a same-length signature sequence from the sample nucleic acid population.

Such a kit may further include right and left PCR primers effective to bind to the adapter binding sites. The tag vector preferably also comprises plasmid DNA for replicating in *E. coli.*

Components of kits for use in preparing same-length signature sequences from a sample nucleic acid population, in accordance with the disclosed methods, may comprise: a first restriction endonuclease which is a type TIs restriction endonuclease having a cleavage site at least 10 nucleotides from its recognition site; a first double stranded adapter containing a recognition site for the second restriction endonuclease; a second adapter having a single stranded overhang effective to hybridize with an overhang produced by cleavage of a double stranded cDNA or cDNA fragment by said second restriction endonuclease, and containing a recognition and cleavage site for a second restriction endonuclease; and the second restriction endonuclease.

The second adapter preferably comprises a set of adapters containing single stranded 2- to 4-nucleotide overhangs, more preferably 2-nucleotide overhangs, of every possible nucleotide sequence. Typically, each adapter contains a primer binding site, and the kit further includes PCR primers effective to bind to these binding sites.

These and other objects and features of the invention will become more fully apparent when the following detailed description of the invention is read in conjunction with the accompanying drawings.

### Brief Description of the Drawings

Figure 1 illustrates one procedure for preparing a library of same-length signature sequences from restriction fragments representing the 3' region of an mRNA sample population, in accordance with an embodiment of the invention;
Figures 2A-B illustrate exemplary procedures for preparing same-length signature sequences from the 3' ends of mRNA, in accordance with further embodiments of the invention;
Figures 3A-C illustrate exemplary procedures for preparing same-length signature sequences from the 5' ends of mRNA, in accordance with further embodiments of the invention;
Figure 4 illustrates an exemplary procedure for preparing same-length signature sequences from fragments representing the 5' region of mRNA, in accordance with a further embodiment of the invention;
Figures 5A-B illustrate a procedure for preparing a solid phase cloned library of signature sequences;
Figure 6 is a flow chart outlining key steps in the preparation of a solid phase cloned library, in accordance with the embodiment of Fig. 1; and
Figure 7 illustrates an alternative procedure for loading tag-signature constructs onto solid phase supports.

### Detailed Description of the Invention

### I. Definitions

The terms below have the following meanings unless indicated otherwise. The term "oligonucleotide", as used herein, includes linear oligomers of natural or modified monomers or linkages, including deoxyribonucleosides, ribonucleosides, anomeric forms thereof, peptide nucleic acids (PNAs), and the like, capable of specifically binding to a target polynucleotide by way of a regular pattern of monomer-to-monomer interactions, such as Watson-Crick type of base pairing, base stacking, Hoogsteen or reverse Hoogsteen types of base pairing. Monomers are generally linked by phosphodiester bonds or analogs thereof to form oligonucleotides ranging in size from a few monomeric units, *e.g.* 3-4, to several tens of monomeric units, *e.g.* 40-60. When an oligonucleotide is represented by a sequence of letters, such as "ATGCCTG," it will be understood that the nucleotides are in 5' → 3' order from left to right, and that "A" denotes deoxyadenosine, "C" denotes deoxycytidine, "G" denotes deoxyguanosine, and "T" denotes thymidine, unless otherwise noted. Usually, oligonucleotides comprise the four natural nucleotides; however, they may also comprise non-natural nucleotide analogs. It is clear to those skilled in the art when oligonucleotides having natural or non-natural nucleotides may be employed; *e.g.*, where processing by enzymes is called for, usually oligonucleotides consisting of natural nucleotides are required.

"Complement" or "tag complement", as used herein in reference to oligonucleotide tags, refers to an oligonucleotide to which a oligonucleotide tag specifically hybridizes to form a perfectly matched duplex or triplex. In embodiments where specific hybridization results in a triplex, the oligonucleotide tag may be selected to be either double stranded or single stranded. Thus, where triplexes are formed, the term "complement" is meant to encompass either a double stranded complement of a single stranded oligonucleotide tag or a single stranded complement of a double stranded oligonucleotide tag.

"Perfectly matched" in reference to a duplex means that the poly- or oligonucleotide strands making up the duplex form a double stranded structure with one other such that every nucleotide in each strand undergoes Watson-Crick basepairing with a nucleotide in the other strand. The term also comprehends the pairing of nucleoside analogs, such as deoxyinosine, nucleosides with 2-amninopurine bases, and the like, that may be employed. In reference to a triplex, the term means that the triplex consists of a perfectly matched duplex and a third strand in which every nucleotide undergoes Hoogsteen or reverse Hoogsteen association with a basepair of the perfectly matched duplex. Conversely, a "mismatch" in a duplex between a tag and an oligonucleotide means that a pair or triplet of nucleotides in the duplex or triplex fails to undergo Watson-Crick and/or Hoogsteen and/or reverse Hoogsteen bonding.

As used herein, "nucleoside" includes the natural nucleosides, including 2'-deoxy and 2'-hydroxyl forms, *e.g.* as described in Kornberg and Baker, DNA Replication, 2nd Ed. (Freeman), San Francisco, 1992. "Analogs", in reference to nucleosides, includes synthetic nucleosides having modified base moieties and/or modified sugar moieties, *e.g.* as described by Scheit, Nucleotide Analogs (John Wiley, New York, 1980); Uhlman and Peyman, Chemical Reviews 90: 543-584 (1990), or the like, with the proviso that they are capable of specific hybridization. Such analogs include synthetic nucleosides designed to enhance binding properties, reduce complexity, increase specificity, and the like.

"Attaching an adapter" to an end of a polynucleotide may refer to ligation of the adapter, preferably in solution phase, or it could comprise employing the adapter in a primer used in the synthesis of the polynucleotide, and thus incorporating the adapter at the end of the polynucleotide.

A fragment "derived from the 5' regions of a source nucleic acid population" preferably includes or consists of DNA that falls within the 5' most fragment of a population of fragments prepared by cleavage of the nucleic acid population, generally by a restriction endonuclease. A fragment "derived from the 3' regions of a source nucleic acid population" can be defined similarly, except that in the case of mRNA and cDNA, the 3' polyA/polyT tract is excluded.

### II. Oligonucleotide Tags

Oligonucleotide "tags" can be used to construct DNA populations attached to solid phase supports, preferably microparticles, for use in the method of the invention. Such tags and methods of their preparation and use are described in detail in PCT Pubn. Nos. WO 96/41001 and WO 96/12014 and in co-owned U.S. Patent No. 5,604,097. As described in the above-cited publications, the tags are selected from minimally cross-hybridizing sets of oligonucleotides. The sequences of any two oligonucleotide tags of such a set always differ by at least two nucleotides, and preferably by three nucleotides. Members of such a set cannot form a duplex or triplex with the complement of another member of the same set with less than two (or three) mismatched nucleotides. Preferably, minimally cross-hybridizing sets comprise subunits that make approximately equivalent contributions to duplex stability as every other subunit in the set. In this way, the stability of perfectly matched duplexes between every subunit and its complement is approximately equal.

A preferred embodiment of minimally cross-hybridizing sets are those whose subunits are made up of three of the four natural nucleotides. As discussed below, the absence of one type of nucleotide in the oligonucleotide tags permits target polynucleotides to be loaded onto solid phase supports by use of the 5'→3' exonuclease activity of a DNA polymerase.

The following is an exemplary minimally cross-hybridizing set of subunits ("words") each comprising four nucleotides selected from the group consisting of A, G, and T:

| | | | | |
|---|---|---|---|---|
| Word: | w₁ | w₂ | w₃ | w₄ |
| Sequence: | GATT | TGAT | TAGA | TTTG |
| Word: | w₅ | w₆ | w₇ | w₈ |
| Sequence: | GTAA | AGTA | ATGT | AAAG |

In this set, each member would form a duplex having three mismatched bases with the complement of every other member.

Oligonucleotide tags for sorting and solid phase cloning may range in length from 12 to 60 nucleotides or basepairs, preferably from 18 to 40 nucleotides or basepairs, and more preferably from 25 to 40 nucleotides or basepairs. Repertoires of single stranded oligonucleotide tags for sorting and solid phase cloning preferably contain at least 100 members; more preferably at least 1000 members; and most preferably at least 10,000 members. As used herein in reference to oligonucleotide tags and tag complements, the term "repertoire" means the total number of different oligonucleotide tags or tag complements that are employed for solid phase cloning (sorting) or for identification. When oligonucleotide tags are used for sorting, they are hybridized to tag complements, which are preferably attached to solid phase supports. Such tag complements can be synthesized on the surface of the solid phase support, such as a microscopic bead or a specific location on an array of synthesis locations on a single support, such that populations of identical, or substantially identical, sequences are produced in specific regions.

Preferably, tag complements are synthesized combinatorially on microparticles, so that each microparticle has attached many copies of the same tag complement. A wide variety of microparticle supports may be used with the invention, including microparticles made of controlled pore glass (CPG), highly cross-linked polystyrene, acrylic copolymers, cellulose, nylon, dextran, latex, polyacrolein, and the like, as known in the art.

Preferably, tag complements in mixtures, whether synthesized combinatorially or individually, are selected to have similar duplex or triplex stabilities to one another, so that perfectly matched hybrids have similar or substantially identical melting temperatures. This feature permits mismatched tag complements to be more readily distinguished from perfectly matched tag complements in the hybridization steps, *e.g.* by washing under stringent conditions.

An exemplary tag library for use in sorting is shown below (SEQ ID NO: 1).

The tag repertoire is represented by [⁴(A,G,T)₈], which denotes eight concatenated four-nucleotide "words", as described above, containing the three nucleotides shown and selected from a minimally cross-hybridizing set, as described above. The flanking regions of the oligonucleotide tag may be engineered to contain restriction sites, as exemplified above, for convenient insertion into and excision from cloning vectors. Optionally, the right or left primers (SEQ ID NOs: 3 AND 2) may be synthesized with a biotin attached (using conventional reagents, *e.g.* available from Clontech Laboratories, Palo Alto, Calif.) to facilitate purification after amplification and/or cleavage. Preferably, for making tag-fragment conjugates, the above library is inserted into a conventional cloning vector, such as pUC 19, or the like. Optionally, the vector containing the tag library may contain a "stuffer" region, "XXX ... XXX," which facilitates isolation of fragments fully digested with, for example, BamHI and BbsI. An exemplary tag vector for solid phase cloning of signatures is shown in Example 1, below (SEQ ID NO: 16).

Sorting and attachment of populations of DNA sequences in a library, *e.g.* a cDNA or genomic library, to microparticles or to separate regions on a solid phase support, is carried out such that each microparticle or region has substantially only one kind of sequence attached; that is, such that the DNA sequences are present in clonal subpopulations.

Preferably, at least ninety-five percent of the DNA sequences have unique tags attached. This condition is achieved by employing a repertoire of tags substantially greater than the population of polynucleotides, as noted further below, and by taking a sufficiently small sample of tagged polynucleotides from the full ensemble of tagged polynucleotides. (It is acceptable that identical DNA sequences have different tags, as it merely results in the same DNA sequence being operated on or analyzed twice.) Such sampling can be carried out either overtly, for example, by taking a small volume from a larger mixture, after the tags have been attached to the DNA sequences; it can be carried out inherently as a secondary effect of the techniques used to process the DNA sequences and tags; or sampling can be carried out both overtly and as an inherent part of processing steps.

Preferably, DNA sequences are conjugated to oligonucleotide tags by inserting the sequences into a conventional cloning vector carrying a tag library, as described above. A sample is taken from this library for amplification and sorting. Sampling may be accomplished by serial dilutions of the library, or by simply picking plasmid-containing bacterial hosts from colonies. After amplification, the tag-DNA conjugates may be excised from the plasmid.

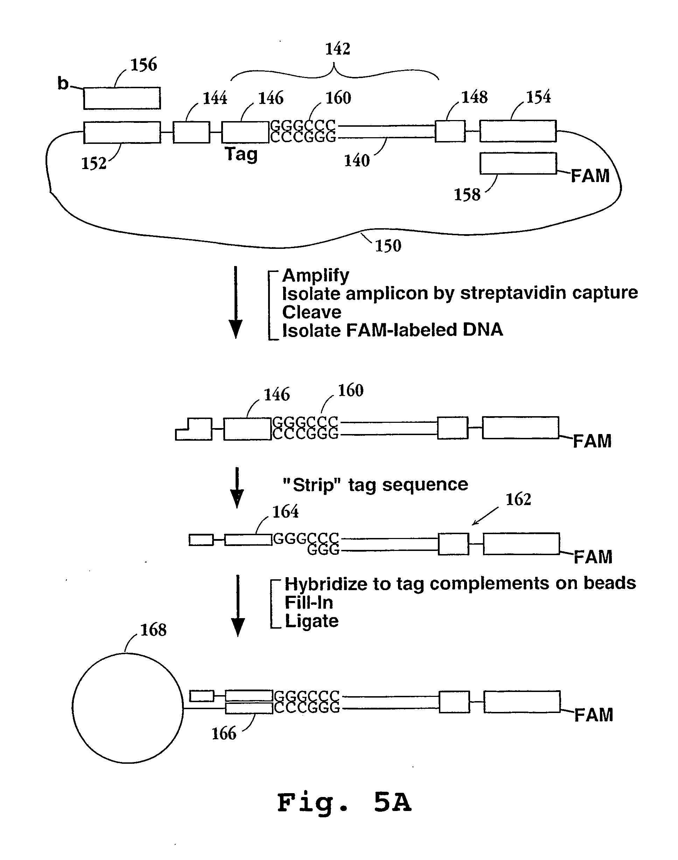

The DNA-tag conjugates are mixed with microparticles containing the tag complements (*e.g.* as shown in Fig. 5A, discussed below) under conditions that favor the formation of perfectly matched duplexes between the tags and their complements. There is extensive guidance in the literature for creating these conditions; see *e.g.* Wetmur, Critical Reviews in Biochemistry and Molecular Biology, 26: 227-259 (1991) and Sambrook et al., Molecular Cloning: A Laboratory Manual, 2nd Edition (Cold Spring Harbor Laboratory, New York, 1989). Preferably, the hybridization conditions are sufficiently stringent so that only perfectly matched sequences form stable duplexes. Under such conditions, the polynucleotides specifically hybridized through their tags may be ligated to the complementary sequences attached to the microparticles. Finally, the microparticles are washed to remove polynucleotides with unligated and/or mismatched tags.

### III. Preparation of Solid Phase Cloned Libraries

### A. Preparation of Signature Sequence Inserts

The signature sequences described herein are generally derived from cDNA. For preparation of a cDNA library, in accordance with conventional methods, mRNA is extracted from each cell or tissue source of interest and converted into cDNA using conventional techniques, as disclosed in, for example, Sambrook et al., Molecular Cloning: A Laboratory Manual, 2nd Edition (Cold Spring Harbor Laboratory, New York); Schena et al., Science 270: 467-470 (1995); DeRisi et al., Science 278: 680-686 (1997). Preferably, the first strand of cDNA is synthesized with a reverse transcriptase in the presence of the four dNTP's, using a primer having a 5' biotin and a poly(dT) region for annealing to the mRNA strands. If desired, the cDNA may be methylated by employing methyl-dCTP in place of dCTP, to prevent unwanted cleavage at later steps by methyl-sensitive restriction endonucleases. Certain other modifications may be employed in accordance with the different protocols for preparing signature sequences described below.

The methods of the invention employ a first adapter having a recognition site for a restriction enzyme whose cleavage site is within the attached cDNA. The most common and widely available of such enzymes are the type IIs restriction enzymes. Other classes of enzymes which fall within this definition include the type IIb restriction enzymes, which have bipartite, interrupted recognition sites, and cleave both DNA strands on both side of the recognition site, which is thus excised from the DNA. Of these enzymes, BcgI, discussed further below in Section F, is commercially available. The type III restriction enzymes have non-palindromic recognition sites, and cleavage occurs approximately 25 bases from the recognition site. The type IIe restriction enzymes include enzymes that would be classified as type IIs but which demonstrate slow or resistant cleavage sites. Efficient cleavage at these sites can be achieved by the binding of another (affector) recognition sequence to a distal, noncatalytic site on the enzyme.

### A1. Signatures from fragments representing 3' ends of cDNA

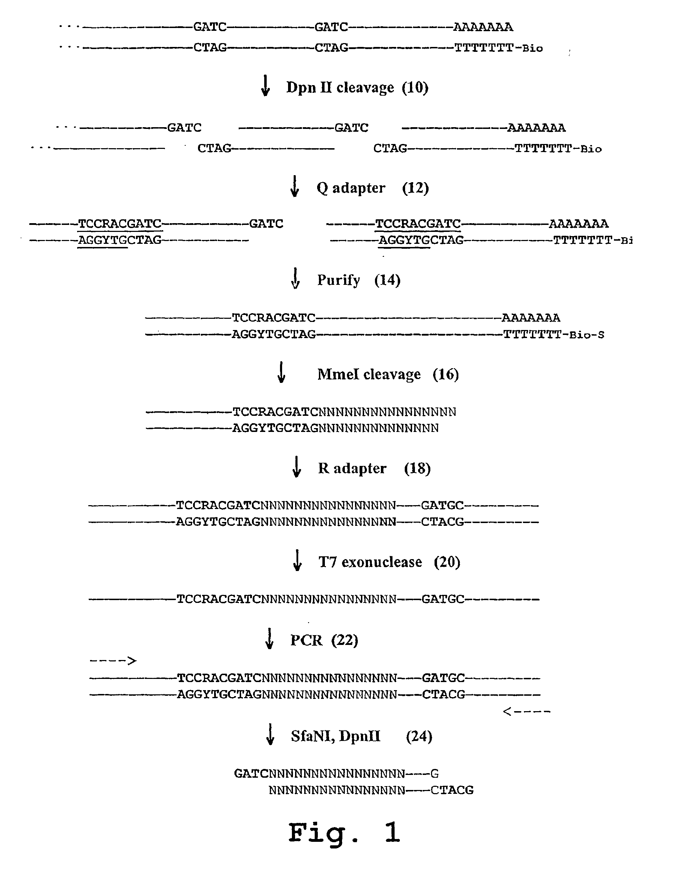

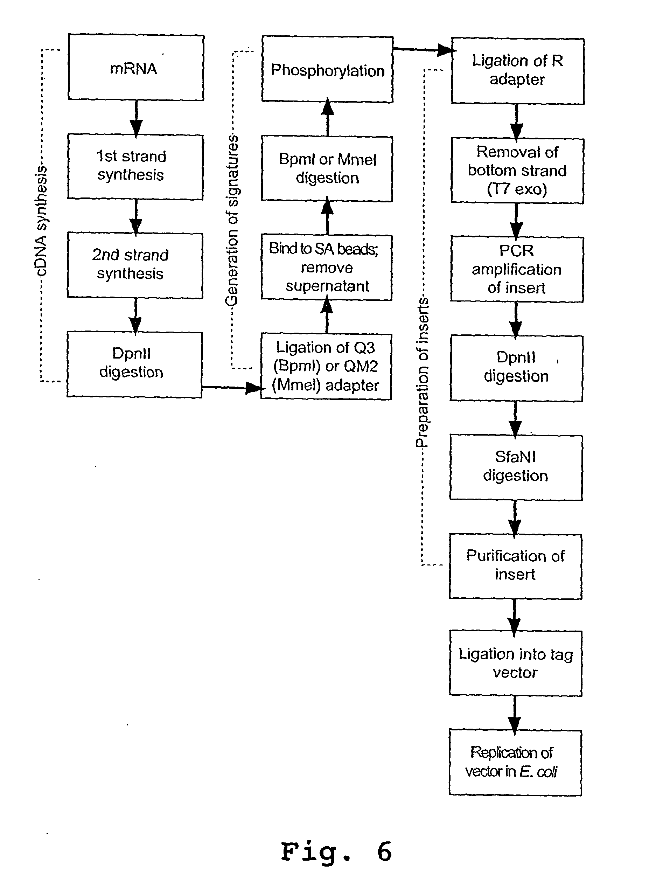

In one embodiment of the method, signature sequences are prepared from cDNA restriction fragments which represent the 3' ends of full length cDNA. An exemplary method is illustrated in Fig. 1. This embodiment is also illustrated in the flow chart shown in Fig. 6 and described in detail in Example 1.

Following second strand synthesis, cDNA is digested (10) with a restriction endonuclease having a two- to four-base recognition site, preferably a four-base recognition site. Suitable endonucleases include, for example, NlaIII, DpnII, ChaI, Csp6I, and isoschizomers thereof. Other suitable enzymes for this and other steps in the processes described herein could be determined by one skilled in the art. This step is effective to produce a population of double stranded fragments having a cleaved end, preferably with a 3' overhang, as shown in Fig. 1.

If desired, at this stage, the 3' overhang may be removed, *e.g.* using T4 DNA polymerase, to produce blunt cleaved ends. As will be apparent from the steps described below, this step results in a longer unique signature sequence. However, for ease of processing, the 3' overhang is generally retained.

A first adapter, termed a Q adapter, is then ligated (12) to the cleaved ends. (Note that regions within the adapters and cDNA in Fig. 1 are generally not shown to scale.) This adapter includes an overhang complementary to that on the cleaved ends, unless the overhang has been removed, in which case a blunt ended adapter is used. The Q adapter also comprises a recognition site for a restriction endonuclease having a cleavage site within the DNA, generally a recognition site for a type IIs restriction endonuclease, having a cleavage site at least 10 nucleotides from its recognition site, and preferably at least 16 nucleotides from its recognition site. Suitable type IIs enzymes include, for example, BmpI, MmeI, Gsul, and isoschizomers thereof.

For example, the adapter of Fig. 1 contains a recognition site for MmeI, having a cleavage site 20 nucleotides in the 3' direction, which is in this case 16 nucleotides into the restriction fragment. The top strand and bottom strand of an exemplary adapter having an MmeI site are shown below, along with a corresponding PCR primer (SEQ ID NOs: 4-6):

### QM2 adapter and primer (for MmeI):

| | | |
|---|---|---|
| QM2primer: | 5'-FAM-CGTTCAGAGTTCTACAGTCCGA | SEQ ID NO: 4 |
| QM2top.S: | | SEQ ID NO: 5 |
| QM2bot.P: | 3'- CAAGTCTCAAGATGTCAGGCTGCTAG-p | SEQ ID NO: 6 |

A second exemplary Q adapter having a partial (see below) Mmel site is shown below, along with a corresponding PCR primer (SEQ ID NOs: 7-8):

| | | |
|---|---|---|
| QPrimer: | FAM-AGACTTCTACGCATCTCCGACA | SEQ ID NO: 7 |
| QAdapter: | | SEQ ID NO: 8 |

The adapters and restriction endonucleases may be selected such that the recognition site of the type IIs endonuclease overlaps the recognition and cleavage site of the enzyme used for initial digestion of the cDNA. For example, when the adapter having SEQ ID NO: 8 above is used with an endonuclease having a recognition site with the first base C (*e.g.* NlaIII, having the recognition site CATG), the recognition site for MmeI (TCCRAC) overlaps this recognition site by one nucleotide. As a consequence, the signature sequence (shown in boldface below) produced upon cleavage, as described further below, extends 17 nucleotides into the fragment, rather than 16 nucleotides (as in the embodiment shown in Fig. 1).

| Q adapter & primer | **Signature** | R adapter & primer |
|---|---|---|
| FAM-AGACTTCTACGCATCTCCGACA | | <- SEQ ID NO: 7 |
| | | |

(The sequence of the Q adapter-signature-R adapter construct above,

A further exemplary Q adapter, having a BpmI site, is shown below, along with a corresponding PCR primer (SEQ ID NOs: 11-12):

### Q adapter and primer (for BpmI):

| | | |
|---|---|---|
| Q3primer: | 5'-FAM-GCTACACGATTCTACAGTCTGGA | SEQ ID NO: 11 |
| Q3top.S: | | |
| Q3bot.P: | 3'-CGATGTGCTAAGATGTCAGACCTCTAG-p | SEQ ID NO: 12 |

The top strand of the Q adapter is protected at the 5' end, *e.g.* by the use of thionucleotides or by a small 5' overhang (as shown for QM2top, above). This prevents digestion of the strand by exonuclease in a later step.

With continuing reference to Fig. 1, the fragment-adapter constructs representing the 3' ends of the cDNA are then bound, via the biotin label, to streptavidin supports (**14**), *e.g.* Dynabeads M-280 (Dynal, Oslo, Norway). This operation serves to isolate fragments representing the 3' ends of the cDNA from other fragments, which do not include a biotin label.

Cleavage with the type IIs restriction endonuclease (**16**) produces cleaved fragments each containing a uniform length, or signature sequence, of the source cDNA. In the embodiment shown in Fig. 1, employing MmeI cleavage, the newly cleaved end has a 2-base 3' overhang. The adapter-signature fragments can then be isolated by eluting from the streptavidin supports.

### A2. Signatures from 3' ends of cDNA

In another embodiment, the polyadenylated region at the 3' end of mRNA is removed, and a Q adapter, as described above, is ligated to the remaining 3' end of the corresponding cDNA.

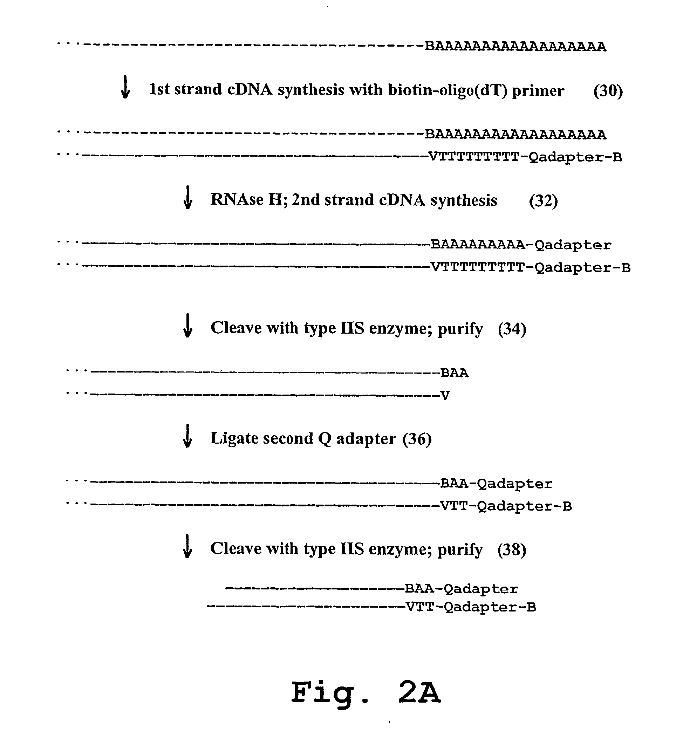

In one method of removing the polyA region, illustrated in Fig. 2A, first strand cDNA synthesis is carried out using an biotin-adapter-oligo(dT) primer (**30**). As described above, the Q adapter typically contains a type IIs recognition site, such that when the Q adapter-cDNA conjugate is cleaved with the respective type IIs enzyme, cleavage occurs within the cDNA, preferably at least six basepairs into the cDNA. A second recognition site may also be included for later cleavage of the Q adapter from the construct, as discussed further below.

Second strand synthesis is carried out (**32**) using 5-methyl cytosine, as above. Cleavage of the double stranded cDNA with the type IIs enzyme **(34)** is effective to remove the polyA region, as shown in Fig. 2A. The cleaved polyA-containing fragments can then be removed via the biotin label **(34).**

A second adapter containing a type IIs recognition site is then ligated **(36)** to the cleaved 3' end of the cDNA. As described above, the enzyme recognizing the recognition site preferably has a cleavage site at least 10 nucleotides from its recognition site, and preferably at least 16 nucleotides from its recognition site. Suitable enzymes include, for example, BmpI, MmeI, GsuI, and isoschizomers thereof. Again, the adapter preferably includes a PCR primer site for amplification and/or a biotin for purification. Cleavage with the type IIs enzyme **(38)** gives the Q adapter linked to a 3' signature.

It can be seen that, in this method, the placement of the recognition sites in the two adapters can be varied to produce cleavage at a desired site, allowing signatures to be captured from different regions near the 3' end of the cDNA.

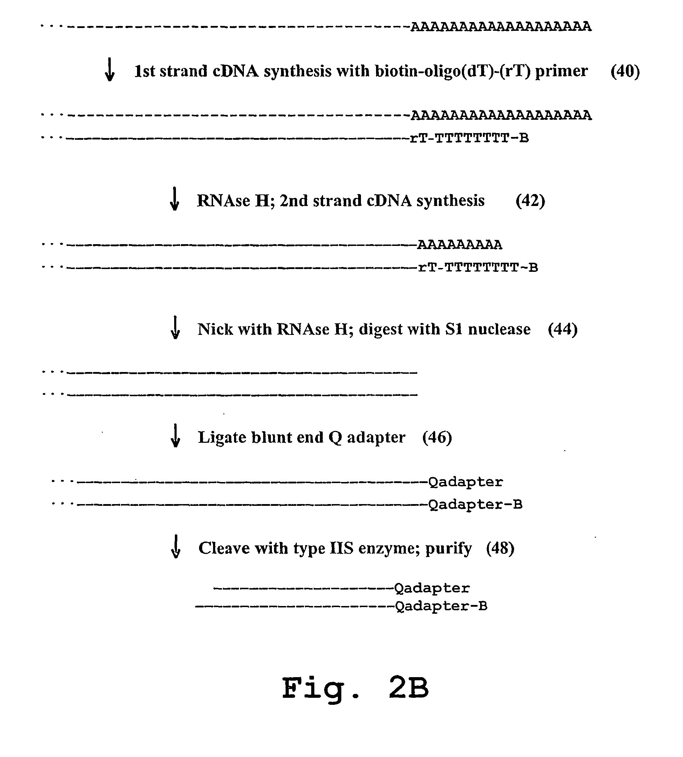

In an alternate procedure for cleaving the polyA tail from the mRNA, illustrated in Fig. 2B, first strand cDNA synthesis **(40)** is primed with a biotin-oligo(dT)-(rT) hybrid primer, having a short sequence of ribonucleotides (rT)ₙ (preferably 1-3 rT's, and more preferably a single rT, although up to about 24 rT's could be used) at or near the 3'-end of the primer. Second strand synthesis**(42)** is carried out using 5-methyl cytosine, as above. The ribonucleotide site of the adapter is then nicked with RNaseH, and the polyA region is digested with S1 nuclease to generate a blunt end **(44).** A blunt end Q adapter, containing a type IIs recognition site, is ligated at the blunt 3' end **(46).** Cleavage with the respective type IIs enzyme **(48)** provides a 3' signature linked to the adapter, which can be purified via a biotin incorporated into the adapter.

### A3. Signatures from 5' ends of cDNA

In another embodiment, signatures are prepared from 5' ends of cDNA, preferably full length cDNA. These 5' signatures can provide sequence information regarding promoter, enhancer, and transcription initiation sites. Combined with information obtained from 3' signatures, 5' sequence information can also facilitate sequencing of full length cDNA, by allowing design of PCR primers which span the complete transcript. Quantitative transcript levels can be determined more accurately.

In addition, derivation of signatures from the ends (either 3' or 5') of full length cDNA, rather than from restriction fragments, allows access to cDNAs which do not contain a given restriction site.

The 5' signatures may be prepared by attaching a Q adapter to the 5' ends of cDNA, typically full length cDNA. As described above, the Q adapter preferably has a type IIs recognition site with a corresponding cleavage site within the cDNA, and may also include a second recognition site for later cleavage of the Q adapter from the construct, as discussed further below. Various methods can be used to attach the adapter to 5' ends of mRNA.

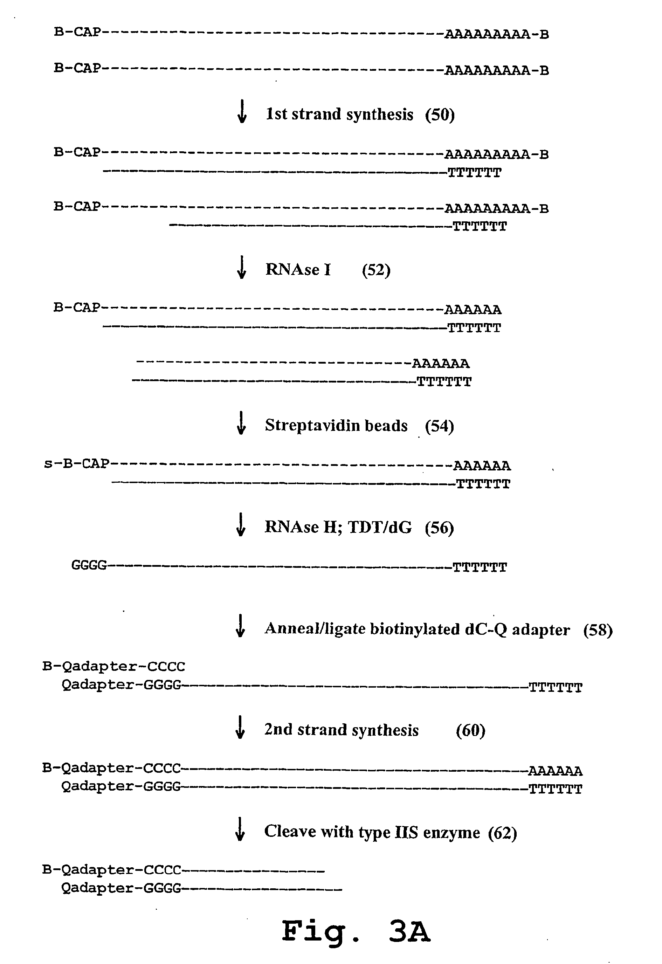

In accordance with one method, illustrated in Fig. 3A, biotin is attached to the 5' end of mRNA, and optionally to the 3' end as well, by utilizing the diol structure present on the 3' and 5' ends of mRNA (see *e.g.* Y. Hayashizaki et al., U.S. Patent No. 6,174,669). First strand cDNA synthesis is carried out **(50)** using an oligo(dT) primer; as above, methyl-dCTP may be employed in place of dCTP, to prevent cleavage of the cDNA by restriction enzymes used in subsequent steps.

The population of DNA/RNA hybrids is then treated with RNAse I **(52),** which cleaves single stranded RNA. As shown in the figure, the 5' biotin is thus retained only for duplexes containing full length mRNA. These duplexes may be isolated by contacting with streptavidin beads **(54).**

The RNA strand is then digested with RNAse H **(56),** and second strand cDNA synthesis is carried out. This can be done in various ways; for example, in one method, the first strand cDNA (now released from the beads) is homotailed at the 5' end with oligo(dG) (or (dC)) using terminal deoxynucleotidyl transferase **(56).** A double stranded Q adapter having an oligo(dC) (or (dG), respectively) overhang, and preferably including elements for later purification, priming, and eventual removal, is then annealed to the 5' end **(58).** Second strand cDNA synthesis is performed **(60)** using DNA polymerase I.

Alternatively, in another method, RNAse H is used to nick the RNA strand in the hybrid for nick-translation second strand synthesis. T4 DNA ligase is then used to seal the fragments primed by the Q adapter-oligo(dC) and nicked RNA.

Cleavage with the type IIs enzyme **(62)** cleaves within the cDNA and gives the adapter linked to a 5' signature, as shown. Preferably, the Q adapter includes a binding moiety such as biotin for purification and/or a primer binding site for amplification.

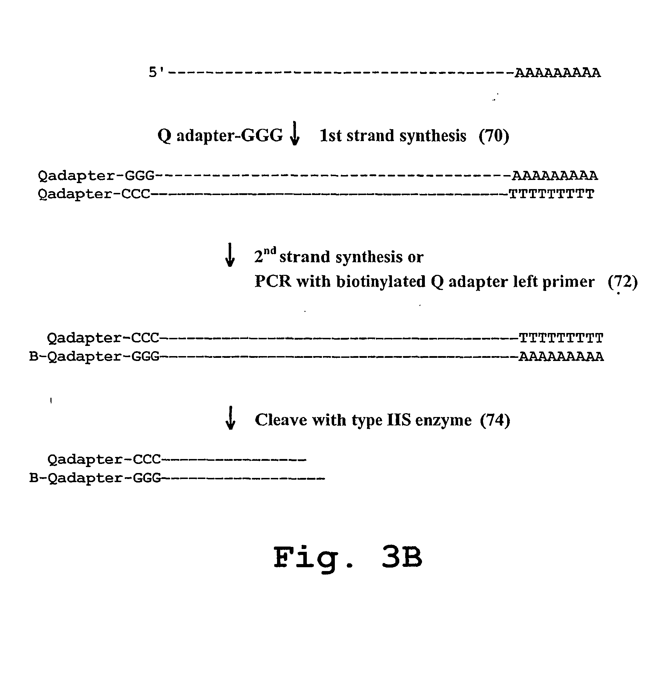

A second method for producing 5' signatures (Fig. 3B), preferably from full length cDNA, utilizes a "template switching oligonucleotide", as described, for example, in A. Chenchik et al., U.S. Patent No. 5,962,272. The template-switching oligonucleotide includes a short dG sequence, for binding to a short dC sequence added to the 3' end of the first strand cDNA by reverse transcriptase, and an adapter having a selected sequence, including *e.g.* a type IIs recognition site and primer site. The oligonucleotide (*e.g.* "Q adapter - GGG" in Fig. 3B) creates an extended template for the reverse transcriptase during first strand synthesis **(70),** which incorporates the adapter sequence at the 3' end of first strand cDNA.

A primer complementary to the primer sequence in the adapter is used for second strand synthesis **(72),** thereby incorporating a Q adapter at the 5' end of the second strand cDNA. Preferably, the Q adapter includes a binding moiety such as biotin for purification. (Alternatively, biotin can be incorporated via the oligo dT primer during first strand synthesis. The use of biotin on either the 3' or 5' end will be determined by the desired purification scheme.) As above, cleavage with the type IIs enzyme **(74)** cleaves within the cDNA and gives the adapter linked to a 5' signature, as shown.

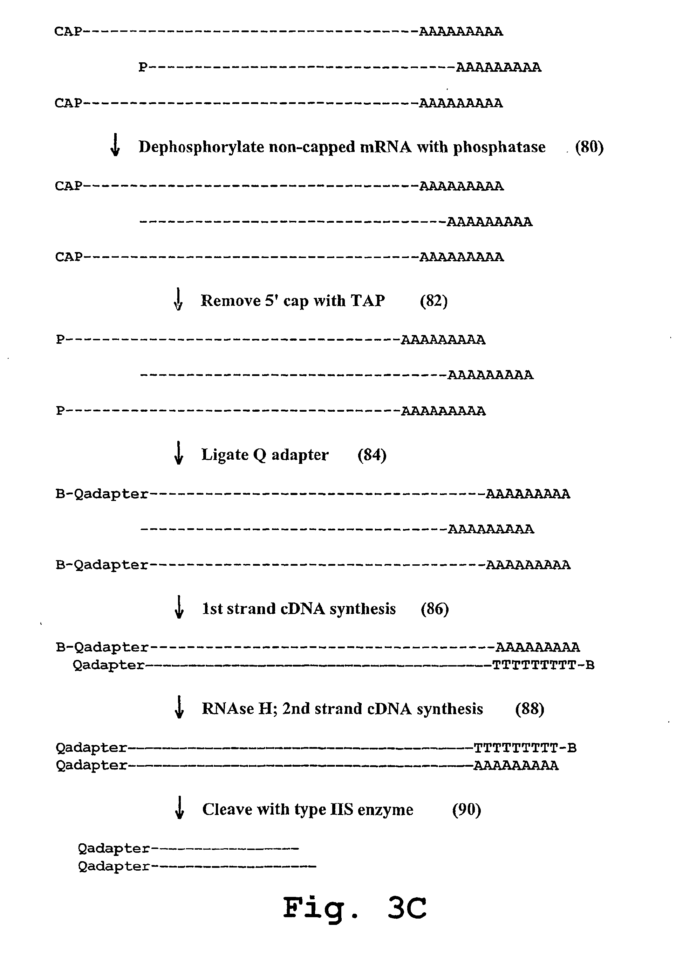

A third method utilizes a modification of a strategy described by K. Maruyama et al. (Gene 138:171-174, 1994) and S. Kato et al. (Gene 150:243-50, 1994) for isolating full length cDNA from preparations that also contain incomplete cDNA transcripts. In accordance with this method (Fig. 3C), any non-capped (non full-length) mRNA species in an mRNA sample are dephosphorylated using calf intestinal phosphatase **(80).** The 5'cap structure of full length mRNA is then removed with tobacco acid pyrophosphatase (TAP), to leave a 5' phosphate **(82).** An adapter containing a type IIs recognition site, and preferably containing biotin for purification, is ligated to these 5' phosphated ends **(84).** At this point, the biotin may be used to isolate these sequences from the non-capped mRNAs.

First strand cDNA synthesis is then carried out **(86),** according to conventional procedures, with a biotinylated oligo(dT) primer and 5-methyl cytosine. The mRNA is removed with RNAse, followed by second strand cDNA synthesis with 5-methyl cytosine **(88).** The double stranded cDNA is then cleaved with the type lIs endonuclease **(90),** to give the Q adapter linked to a 5' signature.

### A4. Signatures from fragments representing 5' ends of cDNA

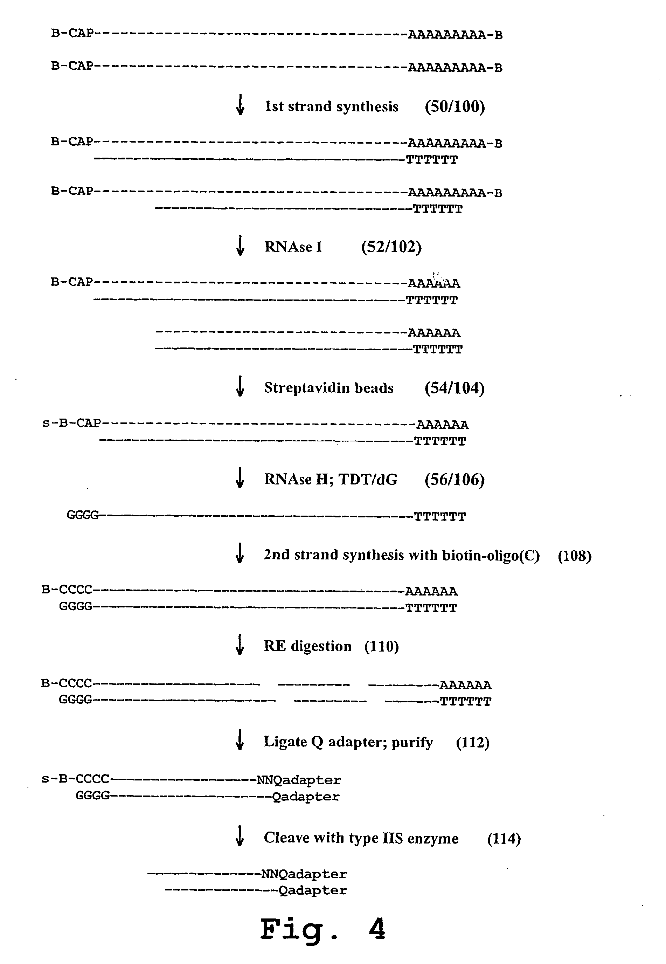

The methods described above, which employ 5' capture of full length cDNA, can also be modified to provide signatures from fragments representing 5' ends of cDNA, by employing steps analogous to steps shown in Fig. 1 for preparing signatures from fragments representing 3' ends of cDNA. An example of a process based on that shown in Fig. 3A is shown in Fig. 4.

The process of Fig. 3A is carried out through the production of 5'-oligo(dG) tailing of first strand cDNA **(56/106).** At this point, second strand synthesis is carried out with a biotin-oligo(dC) primer **(108),** and the double stranded cDNA is digested with a restriction endonuclease **(110),** preferably a 4-cutter endonuclease, and preferably an endonuclease which generates sticky ends (*e.g.* a 2-base or 4-base overhang). Examples include those discussed in Section 1A, *e.g.* NlaIII, DpnII, ChaI, and Csp6I.

A Q adapter, containing a recognition site for a type IIs endonuclease, and designed to anneal to the 3' cleaved ends of the fragments, is then ligated to the fragments **(112).** Fragments representing the 5' ends of the cDNA may then be isolated via the biotinylated primer, *e.g.* on streptavidin beads, as shown **(112).** Cleavage with the type IIs endonuclease **(114)** produces signatures representing the 5' ends of the cDNA linked to the adapter.

### B. Preparation of Cloning Sites

Clonal subpopulations of signature sequences attached to microparticles can be prepared using the processes illustrated in Figs. 5A-B, which are adapted from processes described in, for example, co-owned U.S. Patent No. 6,265,163. As noted above, the present methods provide the advantage of same-length signature sequences (*e.g.* as illustrated at **140** in Fig. 5A) in each clonal subpopulation. The signatures sequences are preferably at least six, and more preferably at least twelve, nucleotides (or basepairs) in length.

With continued reference to Fig. 1, after purification of the signature-Q adapter conjugates, a second adapter, designated an R adapter, is ligated **(18)** to the cleaved end produced by the type IIs endonuclease. Note that the operations described in this and the following section can be applied to same-length signatures prepared according to other embodiments of the invention, as shown in Figs. 2-4. Some modifications may be required, *e.g.* where a cleaved end is a 5' rather than a 3' end, and will be apparent to one skilled in the art.

The R adapter has a single stranded overhang effective to hybridize to an overhang produced by cleavage with the type IIs enzyme. Accordingly, the R adapter is preferably a plurality of adapters containing single stranded overhangs of every possible sequence effective to hybridize with the overhang of the cleaved end (shown in boldface as NN in Fig. 1).

The R adapter also includes a recognition site for a further restriction endonuclease which is preferably also a type IIs enzyme, which preferably produces a 5' extension of at least 2 nucleotides, and more preferably at least 3 nucleotides, upon cleavage. This restriction endonuclease may also be a methyl sensitive endonuclease. Suitable examples include SfaNI, BspMI, BbvI, FokI, BsmFI, BbsI, and isoschizomers thereof.

An exemplary R adapter, having a SfaNI recognition site, is shown below (SEQ ID NOs: 13, 14, and 9, respectively), where NN represents all possible 2-nucleotide sequences.

### R4 adapter and primer:

Rprimer: 3'-TACGAGTTACTACGACTGCCGA

The adapter may also be designed such that dimers of the adapter, formed as side products during ligation of the adapter to the signatures, can be readily cleaved by treatment with an appropriate restriction endonuclease. For example, dimers of the above adapter, having the sequence can be cleaved by the enzyme BsaJI, which has the recognition and cleavage site GGN^NCC.

In a preferred embodiment, to ensure fidelity of complementarity between the top and bottom strands at the NN site, the bottom strand of adapter-signature-adapter construct, which may include mismatches at this site, is removed, *e.g.* by T7 exonuclease **(20).** (This is shown in Fig. 1 for the embodiment described in Section Al, above, and may be applied to signature prepared by any other of the methods described herein.) The top strand is then replicated, preferably by PCR amplification **(22).** Accordingly, the Q and R adapters, in a preferred embodiment, include primer binding sites for PCR, as in the exemplary adapters above. Alternatively, the strand may be replicated by linear primer extension, or by an RNA polymerase, such as T7 polymerase, in which case one of the adapters includes a polymerase binding site.

The constructs are then cleaved **(24)** with the third restriction endonuclease, to cleave the R adapter, and with an endonuclease effective to cleave the construct 5' of the signatures, by cleaving the Q adapter. (For the embodiment shown in Fig. 1, this may be the enzyme initially used for producing cDNA restriction fragments, as discussed below. In other embodiments, the Q adapter may be designed to include a separate restriction site for cleavage from the signature.) This step produces same-length signature sequences (represented by NNNN... in Fig. 1) flanked by cloning sites.

The Q adapter may be designed such that cleavage with the third restriction endonuclease cleaves both adapters, as shown for SEQ ID NO: 8 (Q adapter), incorporated into the Q adapter-signature-R adapter construct (SEQ ID NO: 10) below. The exemplary Q adapter includes a recognition site for the third restriction endonuclease, SfaNI, positioned such that the enzyme cleaves both adapters.

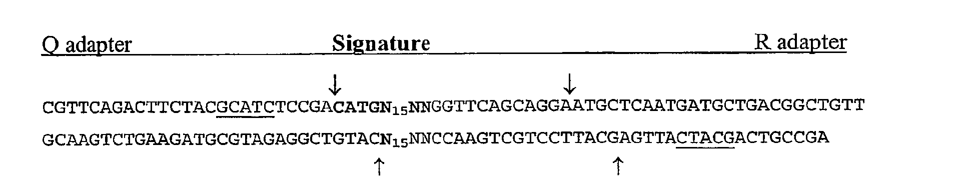

Note that the same-length signature sequences derived from the source polynucleotide may be flanked by some remaining fragment of the first and/or second adapters; however, the overall constructs, including such adapter fragments, are likewise of a uniform length. In general, "signature" as used herein refers to only the sequence derived from the source polynucleotide; "signature fragment" may include one or two short flanking sequences derived from the adapters.

The excised signature-containing fragments are purified using standard techniques, *e.g.* ethanol precipitation and/or electrophoresis. In one embodiment, the PCR amplification of the adapter-signature-adapter construct (penultimate step in Fig. 1) is carried out using biotinylated primers. Following cleavage of the adapters, the signature fragments are purified by elution from a streptavidin support.

### C. Cloning of Tag-Signature Library

After resuspending in an appropriate buffer, the signature fragments are directionally ligated into a library of tag vectors (Fig. 5A), to form a vector library of tag-signature conjugates **(142).** Each tag vector contains a left restriction cleavage site **(144),** an oligonucleotide tag (**146;** see Section I above), a cloning site for insertion of the signature fragment, and a right restriction cleavage site **(148).** Preferably, the vector further comprises plasmid DNA **(150)** for cloning into *E. coli* and primer binding sites **(152, 154)** for later PCR amplification. (Note that a "tag-signature conjugate" as used herein may also include some residual adapter DNA, as noted for "signature fragments" above.)

Preferably, after *E. coli* replication of the library of tag-signature conjugates, a sample of host cells is plated to determine the number of recombinants per unit volume of culture medium. A sample is taken for further processing, the size of the sample depending on the size of tag repertoire (see Section I) used in the tag-vector library. The sample preferably includes a number of conjugates equivalent to about one percent the size of the tag repertoire, in order to minimize the occurrence of "doubles," i.e. two or more conjugates carrying the same tag but different cDNA fragments. (See Brenner et al., PCT Pubn. No. WO 96/41011 and U.S. Pat. No. 5,604,097.) Thus, for a tag repertoire consisting of a concatenation of eight 4-nucleotide "words" selected from a minimally cross-hybridizing set of eight words (see Section III below), the size of the repertoire is 8⁸ or about 1.7 x 10⁷ tags. Accordingly, with such a tag repertoire, a sample of about 1.7 x 10⁵ conjugate-containing vectors is preferably selected for further processing. Practical methods for such sampling are described above and, for example, in U.S. Patent Nos. 6,265,163.

After sampling, the tag-signature conjugates are preferably amplified by PCR, using a biotinylated primer **(156)** and a labeled primer **(158),** in the presence of 5-methyl dCTP, after which the resulting amplicon is isolated by streptavidin capture. Restriction site **(144)** in the vector preferably corresponds to a rarecutting restriction endonuclease, *e.g.* PacI, NotI, FseI, Pmel, or SwaI, which permits the captured amplicon to be released from a support with minimal probability of cleavage occurring at a site internal to the signature fragment.

Where the tag-signature conjugates are loaded from the replicated vectors without PCR amplification, the conjugates are released from the vectors and isolated, *e.g.* by electrophoretic purification. Preferably, an adapter having a fluorescent label is ligated to the construct, for later use in FACS sorting, as described below.

### D. Loading onto Solid Phase Supports

A "stripping" reaction is carried out to render the tags of the tag-signature conjugates single stranded (see *e.g.* Brenner, U.S. Pat. No. 5,604,097). This can be accomplished, for example, by using a DNA polymerase having 3'→5' exonuclease activity, preferably T4 DNA polymerase, in the presence of a single dNTP. Junction **(160),** shown in Fig. 5A, has the sequence 5'-GGGCCC-3' (top strand), and causes the stripping reaction to be halted at the G triplet when the exonuclease reaction is performed in the presence of dGTP. In addition, the tags are designed to contain only three of the four natural nucleotides, as discussed in Section I, in this case only A's, C's, and T's. Thus, when the released tag-signature conjugates are treated with T4 DNA polymerase in the presence of dGTP, the complementary strands of the tags are stripped away to the first G, as shown in Fig. 5A.

When the "stripping" reaction is quenched, the result is duplex **(162)** with single stranded tag **(164).** After isolation, the following steps are implemented (see *e.g.* U.S. Patent No. 5,604,097): the tag-signature conjugates are hybridized to tag complements **(166)** attached to microparticles **(168);** a fill-in reaction is carried out to fill any gap between the complementary strand of the tag-signature conjugate and the 5' end of the tag complement attached to the microparticle, and the complementary strand of the tag-cDNA conjugate is ligated to the 5' end of tag complement **(166),** which is phosphorylated for this purpose.

Because a sampled set of tag-signature conjugates (as described above) is hybridized to a full repertoire of tag complements, the conjugates will generally hybridize to only about one percent of the microparticles. Loaded microparticles can be separated from unloaded microparticles by use of a fluorescence-activated cell sorter (FACS), which detects the fluorescent label on the amplified conjugates, attached by way of the PCR primer **(158).**

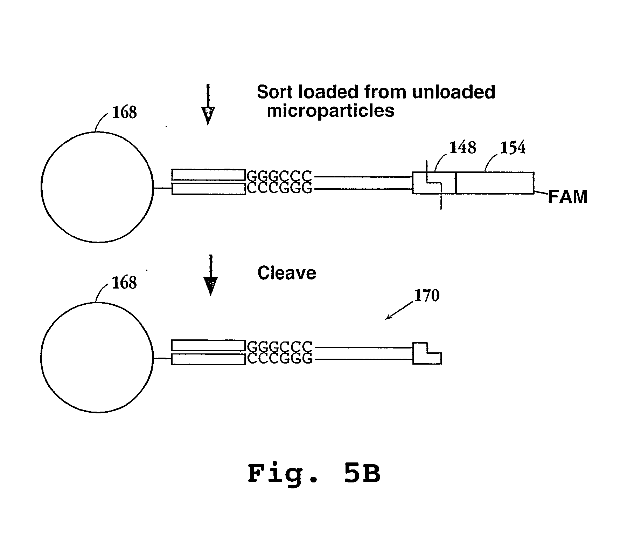

Prior to sequencing, the fluorescent label is preferably removed, by treatment with a restriction endonuclease recognizing cleavage site **(148)** of the vector (Fig. 5B). This cleavage results in microparticle **(168)** with double stranded tag-signature conjugate **(170).**

### E. Alternative Loading Method

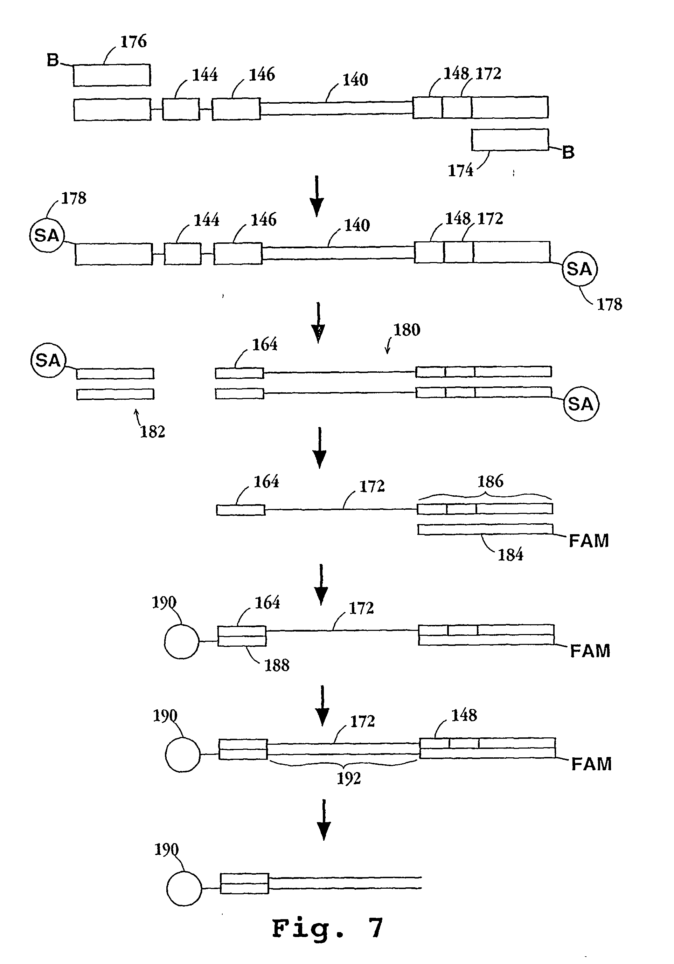

An alternative method of loading the constructs onto beads, which does not require a "chewback" reaction, can be carried out as follows (Fig. 7). In this embodiment, the first adapter above preferably includes a further restriction site **(172), 3'** to the type IIs restriction site, for later primer removal, as described below.

The vectors containing the tag-signature conjugates are linearized, *e.g.* by cutting with PacI. The top strands are replicated, if desired, by *in vitro* transcription, then the bottom strands are reverse transcribed using a first biotin-labeled primer **(174),** and second strand synthesis of the top strands is carried out using a second biotin-labeled primer **(176).** The vectors are then cleaved at the left restriction cleavage site, thereby removing the first biotin labels, and the product is loaded onto streptavidin beads **(178),** thus binding the second biotin labels. The top strands **(180)** are then eluted from the support. (A short 3' region **(182)** of the bottom strand will also be eluted but should not interfere with further processing).

A primer **(184),** preferably fluorescently labeled for FACS sorting of loaded beads, is annealed to the region **(186)** of each top strand which is 3' of the signature. The top strands, bearing a single stranded tag and signature, are then contacted with a library of tag complements **(188),** each on a separate solid phase support **(190),** as above, thereby hybridizing the tags to their respective complements.

The primer is then extended to replicate the bottom strand **(192)** of the signature, which is then ligated to the tag complement on the solid support. The above-referenced further restriction site can then be cleaved to remove the primer label and the portion of the DNA 3' to the signature.

### F. Alternative Methods for Preparing Same Length Signatures

In one method, signatures adjacent to methylated cytosine in genomic DNA can be captured. Sequence data from such signatures could be used to map methylated CpG sites, such as methylated promoter regions, in genomic DNA. About 60-90% of CpG sites in vertebrate DNA are estimated to be methylated (Bird, A.P., Nature 321:209-213, 1986).

According to this method, the DNA is cleaved with the enzyme McrBC, which recognizes the sequence 5'...Pu^{m}C(N40-3000)Pu^{m}C....3', where optimally N = 55-103 bases, and ^{m}C represents methylated cytosine. The enzyme cleaves the DNA between each pair of "half-sites", close to one half-site or the other. Cleavage positions are distributed over several base pairs, approximately 30 base pairs from the methylated base (Stewart, F.J. and Raleigh, E.A., Biol. Chem. 379, 611-616, 1998).

Cleavage, which is believed to produce blunt ended fragments, is followed by ligation of a blunt ended adapter having a type IIs recognition site, as described above. Subsequent steps (cleavage with type IIs enzyme, ligation of "R" adapter, cloning, etc.) are as described above.

In another method, same-length signatures are generated in one step, by cleavage with a "double cutting" enzyme such as the type IIb enzyme BcgI, which recognizes the sequence 5'... (N)₁₀CGA(N)₆TGC(N)₁₂... 3', or the enzyme BaeI, which recognizes the sequence 5'...(N)₁₀AC(N)₄GTAYC(N)₁₂...3' and produces 5-nucleotide overhangs. Cleavage thus generates a 32 base pair or 33 base pair signature sequence, respectively, to which adapters can be ligated for cloning.

### IV. Sequencing of Signatures

The disclosure provides, in one embodiment, a method of massive parallel analysis of expressed genes, allowing detection and isolation of differentially expressed sequences, without requiring prior knowledge of the differentially expressed genes being monitored. More generally, the method allows detection of differentially represented nucleic acids from any two nucleic acid populations, such as variations in genomic DNA. The method can be used for analyzing relative gene expression in a plurality of cells and/or tissues; *e.g.* a diseased tissue or cell type and a healthy tissue or cell type, or a cell or tissue type being subjected to a stimulus or stress, *e.g.* a change of nutrients, temperature, or the like, and the corresponding cell or tissue type in an unstressed or unstimulated state. The method can also be used for identifying differentially represented variations in genomic DNA among individuals, *e.g.* SNP's, deletions, insertions, or duplications.

A benefit of the method is that, in many cases, the identity of the nucleic acids being analyzed need not be known prior to analysis. However, in some cases, prior knowledge of the expected location of the differentially represented sequence, *e.g.* a SNP in genomic DNA, is useful.

The solid phase cloned signatures can be sequenced by any of a number of stepwise sequencing methods which sequentially identify nucleotides, or short sequences of nucleotides, along the length of the support-bound sequence. Such sequencing methods include those described, for example, in Brenner, U.S. Patent Nos. 5,604,097, 5,962,228, and 5,599,675. Conventional sequencing methods, including sequencing by hybridization (SBH) and sequencing by synthesis, can also be used.

In one embodiment, the sequences are identified simultaneously in parallel by MPSS (massively parallel signature sequencing), as described below and in Brenner, U.S. Pat. No. 6,013,445 and Albrecht et al., PCT Pubn. No. WO 97/46704. This procedure is preferably carried out with the following steps:
(a) ligating an encoded adapter to an end of a fragment on a microparticle, the encoded adapter having a nuclease recognition site of a nuclease whose cleavage site is separate from its recognition site;
(b) identifying one or more nucleotides at the end of the fragment by the identity of the encoded adapter ligated thereto;
(c) cleaving the fragment with a nuclease recognizing the nuclease recognition site of the encoded adapter such that the fragment is shortened by one or more nucleotides; and
(d) repeating said steps (a) through (c) until a desired number of nucleotides at the end of the fragment are identified.

Each encoded adapter of step (a) has a protruding strand and an oligonucleotide tag selected from a minimally cross-hybridizing set of oligonucleotides, as discussed in Section I and further in Albrecht et al., PCT Pubn. No. WO 97/46704. Encoded adapters whose protruding strands form perfectly matched duplexes with the complementary protruding strands of a fragment are ligated. After ligation, the identity and ordering of the nucleotides in the protruding strand is determined, or "decoded," by specifically hybridizing a labeled tag complement, or "decoder", to its corresponding tag on the ligated adapter. Preferably, the length of single stranded tag complements for delivering labels is between 8 and 20, more preferably between 9 and 15.

In the identification step, successive sets of tag complements, or decoders, are specifically hybridized to the respective tags carried by the ligated encoded adapters. The type and sequence of nucleotides in the protruding strands of the polynucleotides are identified by the label carried by the specifically hybridized decoder and the set from which the decoder came, as described in U.S. Patent No. 5,599,675.

### EXAMPLES

The following examples illustrate but are not intended in any way to limit the invention.

### Materials

Oligonucleotides were purchased or synthesized by conventional procedures. Rapid Ligation Buffer 1, Rapid Ligation Buffer 2, and Rapid Ligation Ligase are components of the Rapid DNA Ligation Kit, Roche Biochemical #1635379. Reagents having the designation NEB are supplied by New England Biolabs, Beverly, MA

### Preparation of Cloned Signature Library from cDNA

### A. Digestion with first Restriction Endonuclease

cDNA was prepared from approx. 1 pg mRNA using conventional protocols, precipitated with EtOH, and cleaved with DpnII as follows. To the pellet was added 10 µl 10 × *Dpn*II buffer and 1.5 µl *Dpn*II (50 U/µl), and the mixture was incubated at 37°C for 2 hours. The mixture was then diluted with water and extracted with 200µl buffer-saturated phenol (2x) and 200µl chloroform:isoamyl alcohol (24:1), followed by addition of 20µl 3M NaOAc and 500µl-20° EtOH, and incubated at -20°C overnight. The pellet was washed with -20° 70% EtOH.

### B. Generation of adapters

Oligonucleotides designated Q3top.S, Q3bot.P, QM2top.S, QM2bot.P, R4top.FAM and RbotAA...TT (see below) were suspended at 200 µm each in water. For each adapter, 30 µl "top" oligo, 30 µl "bottom" oligo, 10 µl 10 × NEB2 and 30 µl water were mixed in a 0.5 ml Eppendorf tube, heated in a 95° block for 5 minutes, allowed to cool to room temperature, spun briefly, and stored at -20°C.

### Oligonucleotides:

Q3top.S: 5'-GsCsTsAsCsACGATTCTACAGTCTGGA (SEQ ID NO: 11)
Q3bot.P: 5'-p-GATCTCCAGACTGTAGAATCGTGTAGC (SEQ ID NO: 12)
Q3primer.FAM: 5'-FAM-GCTACACGATTCTACAGTCTGGA (SEQ ID NO: 11)
QM2top.S: 5'-CsGsTsTsCsAGAGTTCTACAGTCCGAC (SEQ ID NO: 5)
QM2bot.P: 5'-p-GATCGTCGGACTGTAGAACTCTGAAC (SEQ ID NO: 6)
FAM.QM2primer: 5'-FAM-CGTTCAGAGTTCTACAGTCCGA (SEQ ID NO: 4) R4botNN: 5'-AGCCGTCAGCATCATTGAGCATTCCTGCTGAACCNN (SEQ ID NO: 14)
Rprimer.FAM: 5'-FAM-AGCCGTCAGCATCATTGAGCAT (SEQ ID NO: 9)
s = phosphorothioate linkage; p = 5'-phosphate; FAM = 6-fluorescein amidite

### C. Ligation of Q adapter and binding to SA beads

To the mixture of *Dpn*II-fragments from A, above, and 9.5 µl Q adapter (Q3 or QM2) was added 2.3 µl Rapid Ligation Buffer 2. The mixture was mixed and spun briefly, followed by addition of 11.8µl Rapid Ligation Buffer 1, further mixing, addition of 1 µl Rapid Ligation Ligase, further mixing, and incubation for 4 h at room temperature. The mixture was then treated with 80 µl 5M NaCl and 196 µl TE (100mM NaCl, 20mM Tris-HCl (pH 7.5), 10mM EDTA), and heated at 65°C for 10 min to inactivate the ligase.

Following addition of 4 µl BSA (10 mg/ml), the mixture was added to 150 µl Streptavidin-Dynabeads M-280 (Dynal 112.05) which had been washed 2x in 500µl TE (see above) and resuspended in 100µl TE. The mixture was incubated 1h at 30°C under permanent rotation. The supernatant was withdrawn and the beads washed carefully with 5× 500 µl TE.

### D. Cleavage with type IIs Restriction Endonuclease (removal of signatures from SA beads)

A second digest was prepared using either BpmI or MmeI, depending on which adapter (Q3 or QM2, respectively) was used above. The BpmI digest contained 40µl 10 × NEB3 buffer, 10µl BSA (10 mg/ml), 6 µl *Bpm*I (2U/µl), and water to 400 µl. The MmeI digest contained 40µl 10 × *Mme*I buffer, 40µl 10 × SAM (400 µM), 10µl BSA, 8µl *Mme*I (4U/µl), and water to 400 µl.

The digest was mixed with the SA beads and incubated under rotation at 37°C for 1.5 h, then transferred to a magnetic particle concentrator for removal of supernatant. The mixture was dephosphorylated by addition of 3 µl shrimp alkaline phosphatase (SAP; 1 U/µl; Amersham), which, after 1 h at 37°C, was inactivated by incubating at 70°C for 20 min. The mixture was cooled and extracted with buffer-equilibrated phenol (100 µl) and CHCl₃:IA.A (24:1) (100 µl). After addition of 0.1 vol. 3M NaOAc (pH 5.2) and 1 µl glycogen (20µg/µl), the mixture was precipitated with 2.5 vol. ethanol.

### E. Ligation of second (R) adapter and removal of bottom strand

The pellet was resuspended in R4 adapter ligation mix, consisting of 1 µl R4 adapter (60 µM, see above) and 3 µl H₂O. The following were added in sequence, with mixing/spinning after each addition: 1 µl Rapid Ligation Buffer 2 (5×); 5 µl Rapid Ligation Buffer 1 (2×); and 1 µl Rapid Ligation Ligase. The mixture was incubated 4h at room temperature, followed by addition of 9 µl 10 × NEB4 and 79 µl H₂O, at which point it was immediately heated to 65°C for 10 min to inactivate the ligase.

After quenching the mixture on ice, 2 µl T7 Exonuclease (10 U/µl) (New England Biolabs #M0263S) was added, followed by water to 100 µl total volume. The mixture was incubated for 30 min. at 37°C, then inactivated at 95°C for 5 min.

### F. Generation of inserts (PCR amplification; removal of adapters)

The amplification mixture was prepared as follows:

| | |
|---|---|
| 10 µl | Exo-treated DNA, above |
| 10 µl | 10 × cloned Pfu Buffer |
| 4 µl | 10 µM Q3primer.FAM or QM2primer.FAM |
| 4 µl | 10 µM Rprimer.FAM |
| 2 µl | SC dNTP mix (10 mM each dATP + dGTP + dTTP + 5-Me-dCTP) (Note: 5-Me-dCTP is used in both first strand and second strand.) |
| 68 µl | H₂O |
| 2 µl | Pfu Turbo Hotstart polymerase (2.5U/µl) (Stratagene) |
| Water to | 100 µl |

### Amplification was carried out using the following cycle:

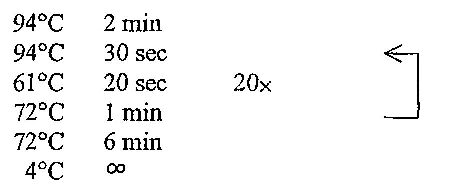

The reaction mixture was extracted with phenol and with chloroform, followed by addition of 1 µl glycogen, 50 µl 7.5M NH₄OAc, and 400 µl ethanol, and precipitation on CO₂(s) for 30 minutes or at -20°C overnight.

The pellet was washed with ethanol and resuspended in 12.5 µl water and 1.5 µl 10 × *Dpn*II-buffer, followed by addition of 1 µl *Dpn*II (NEB, 10 U/µl) and incubation at 37°C for 1-1.5 h. To the restriction reaction was added 13.5 µl NEB3 and 120.5 µl H₂O followed by inactivation at 65 °C for 20 min.

SfaNI (NEB; 1 U/µl) was added (6 µl), followed by incubation at 37°C for 1-1.5 h. The mixture was extracted once with phenol:chloroform:IAA (25:24:1), and 16 µl 3M NaOAc and 450 µl ethanol (-20°) were added. The pellet was washed and then resuspended in 12 µl TE (see above).

### G. Electrophoretic purification

Loading buffer (50% glycerol + BPB; 4 µl) was added to the suspension from F, and the mixture was loaded onto 20% PAGE/1×TBE 10-well gel (Novex) and electrophoresed for 40 minutes at 200V. The relevant bands (29 bp for *Bpm*I, 32 bp for *Mme*I) were identified and isolated by conventional procedures.

### H. Cloning the library

A ligation mixture was prepared, consisting of 1 µl vector (MBS1-8-word tag/BamHI/Bbsl/EcoRV/CIAP/gel purified, ∼200 ng/µl; SEQ ID NO: 16 below), 2 µl Rapid Ligation Buffer 2 (5×), and 6 µl insert (above). (The oligonucleotide tags are cloned into the BseRI-Bsp120I site of the vector.) The mixture was spun briefly, followed by addition of 10 µl Rapid Ligation Buffer 1 (2×), further mixing/spinning, and 1 µl Rapid Ligation Ligase. The mixture was incubated at room temperature for 4 hours.

After ligation, 10 µl 3M NaOAc and 70 µl H₂O were added, and the mixture was extracted once with phenol:chloroform:IAA (25:24:1), followed by addition of 2 µl Pellet Paint^{®} (Novagen) and 275µl-20° ethanol (100%). The mixture was incubated on CO₂(s) for at least 30 minutes and spun 10 minutes. The supernatant was removed and the pellet washed with -20° ethanol (70%). After further spinning and removal of supernatant, the pellet was resuspended in 10 µl H₂O.

An electrocompetent *E. coli* strain, TOP10 (Invitrogen), was transformed with the vector, using 0.5 µl ligation product and 40 µl cells, according to standard procedures.

## Claims

1. A method of preparing a library of same-length signature sequences from a source nucleic acid population, the method comprising:
(a) attaching to an end of each of a population of polynucleotides, a first adapter containing a recognition site for a first restriction endonuclease, such that the cleavage site for the endonuclease is within the polynucleotide, wherein the first restriction endonuclease is a type IIs restriction endonuclease having a cleavage site at least 16 nucleotides from its recognition site, and wherein the end to which the adapter is attached is the same for each polynucleotide, and is selected from: (i) the 5' end of a full length cDNA transcript, (ii) the 3' end of a cDNA transcript from which the polyA/polyT tract has been removed, (iii) the 5' end of a cDNA fragment produced by cleavage of cDNA with a restriction endonuclease, and (iv) the 3' end of a cDNA fragment produced by cleavage of cDNA with a restriction endonuclease;
(b) cleaving the polynucleotides with the first restriction endonuclease, to produce a population of adapter-signature conjugates, each containing a same-length signature sequence of source nucleic acid, at least six basepairs in length, having a newly cleaved end; and
(c) ligating to the newly cleaved ends of said signatures in said adapter-signature conjugates a second adapter, said second adapter containing one or more of (i) a primer binding site and (ii) a recognition and cleavage site for a second restriction endonuclease, to produce a library of adapter-signature-adapter constructs.

2. The method of claim 1, further comprising the step of (d) digesting the constructs with the second endonuclease and a restriction endonuclease effective to cleave the first adapter, to produce a library of same-length signature fragments flanked by cloning sites.

3. The method of claim 1 or 2, wherein attaching step (a) is carried out in solution phase.

4. The method of any one of claims 1 to 3, wherein the end to which the first adapter is attached is selected from: (i) the 5' end of a full length cDNA and (ii) the 3' end of a full length cDNA from which the polyA tract has been removed.

5. The method of any one of claims 1 to 3, wherein the end to which the first adapter is attached is selected from: (iii) the 5' end of a cDNA fragment produced by cleavage of cDNA with a restriction endonuclease, and (iv) the 3' end of a cDNA fragment produced by cleavage of cDNA with a restriction endonuclease.

6. The method of claim 5, wherein a portion of said cDNA fragments of (iii) are derived from the 3' regions of said source nucleic acid population, and a portion of said cDNA fragments of (iv) are derived from the 5' regions of said source nucleic acid population.

7. The method of claim 6, wherein said fragments representing the 3' regions or the 5' regions of said source nucleic acid population are isolated from other cDNA fragments following said attaching.

8. The method of claim 2, wherein at least one adapter includes a binding site for a primer or polymerase, and the method further comprises, following step (c) and preceding step (d):
removing the bottom strand of each adapter-signature construct; and
regenerating the bottom strand, by reverse transcription, primer extension, or PCR amplification.

9. The method of claim 1, wherein the first restriction endonuclease is selected from Bpml, Mmel, Gsul, and isoschizomers thereof.

10. The method of any one of claims 1 to 9, wherein said signatures are at least ten basepairs in length.

11. The method of claim 2, further comprising the steps of:
(e) attaching an oligonucleotide tag to each signature fragment, such that substantially all different signature fragments have different oligonucleotide tags attached, to form tag-signature conjugates;
(f) contacting the tag-signature conjugates with a library of tag complements, each on a separate solid phase support, and hybridizing the tags to their respective complements, to form solid-phase supported clonal subpopulations of signature sequences; and
(g) sequencing a plurality of the solid-phase supported signature sequences.

12. The method of claim 11, wherein attaching said tags comprises:
(i) ligating the signature fragments into a library of oligonucleotide tag-vectors,
wherein each tag-vector comprises: a left restriction cleavage site, an oligonucleotide tag, a cloning site for insertion of the signature fragment, and a right restriction cleavage site, to form a vector library of tag-signature conjugates; and
(ii) replicating said vector library in a host organism.

13. The method of claim 12, wherein the number of different oligonucleotide tags in the tag-vector library is greater than the number of different fragments by a factor of at least 100, and further comprising the step of taking a sample from the vector library, such that substantially all different polynucleotide fragments within the sample have different oligonucleotide tags attached.

14. The method of claim 12, further comprising:
(iii) cleaving said tag-signature conjugates from the vector library;
(iv) removing the bottom strand of the tag component of the tag-signature conjugates;
(v) contacting the tag-signature conjugates with a library of tag complements, each on a separate solid phase support, thereby hybridizing the single stranded tags to their respective complements; and
(vi) ligating the bottom strands of the signature fragments to the tag complements;
thereby forming a library comprising solid-phase supported clonal subpopulations of each signature sequence from the source polynucleotide population.

## Patentansprüche

1. Verfahren zur Herstellung einer Bibliothek von Erkennungssequenzen derselben Länge aus einer Ausgangs-Nukleinsäurepopulation, wobei das Verfahren umfasst:
(a) Anbringen eines ersten Verbindungsstücks, enthaltend eine Erkennungssequenz-Stelle für eine erste Restriktionsendonuklease, an ein Ende einer jeden Population von Polynukleotiden, so dass die Spaltstelle für die Endonuklease innerhalb des Polynukleotids liegt, wobei die erste Restriktionsendonuklease eine Typ Ils-Restriktionsendonuklease mit einer Spaltstelle darstellt, die mindestens 16 Nukleotide von ihrer Erkennungssequenz-Stelle entfernt liegt, und wobei das Ende, an welchem das Verbindungsstück angebracht wird, für jedes Polynukleotid dasselbe darstellt, und wobei das Ende ausgewählt ist aus: (i) dem 5'-Ende eines Volllängen-cDNA-Transkripts, (ii) dem 3'-Ende eines cDNA-Transkripts, aus welchem der polyA/polyT-Strang entfernt worden ist, (iii) dem 5'-Ende eines cDNA-Fragments, das durch Spaltung von cDNA mit einer Restriktionsendonuklease erzeugt worden ist, und (iv) dem 3'-Ende eines cDNA-Fragments, das durch Spaltung von cDNA mit einer Restriktionsendonuklease erzeugt worden ist;
(b) Spalten der Polynukleotide mit der ersten Restriktionsendonuklease, um eine Population von Verbindungsstück-Erkennungssequenz-Könjugaten zu erzeugen, die jeweils eine Erkennungssequenz derselben Länge der Ausgangs-Nukleinsäure enthalten, die mindestens sechs Basenpaare lang ist, und ein neu gespaltenes Ende aufweisen; und
(c) Verbinden eines zweiten Verbindungsstückes mit den neu gespaltenen Enden der Erkennungssequenzen in den Verbindungsstück-Erkennungssequenz-Konjugaten, wobei das zweite Verbindungsstück mindestens eine (i) Primer-Bindungsstelle und (ii) eine Erkennungssequenz- und Spaltstelle für eine zweite Restriktionsendonuklease enthält, um eine Bibliothek von Verbindungsstück-Erkennungssequenz-Verbindungsstück-Konstrukten zu erzeugen.

2. Verfahren nach Anspruch 1, weiterhin umfassend den Schritt (d) Verdauen des Konstrukts mit der zweiten Endonuklease und einer Restriktionsendonuklease, die das erste Verbindungsstück wirksam spaltet, um eine Bibliothek von Erkennungssequenz-Fragmenten derselben Länge zu erzeugen, die von Klonierungsstellen flankiert sind.

3. Verfahren nach Anspruch 1 oder 2, wobei der Schritt des Anbringens (a) in Lösung durchgeführt wird.

4. Verfahren nach einem der Ansprüche 1 bis 3, wobei das Ende, an welches das erste Verbindungsstück angebracht wird, ausgewählt ist aus: (i) dem 5'-Ende einer Volllängen-cDNA und (ii) dem 3'-Ende einer Volllängen-cDNA, aus welcher der polyA-Strang entfernt worden ist.

5. Verfahren nach einem der Ansprüche 1 bis 3, wobei das Ende, an welches das erste Verbindungsstück angebracht wird, ausgewählt ist aus: (iii) dem 5'-Ende eines cDNA-Fragments, das durch Spaltung von cDNA mit einer Restriktionsendonuklease erzeugt wurde und (iv) dem 3'-Ende eines cDNA-Fragments, das durch Spaltung von cDNA mit einer Restriktionsendonuklease erzeugt wurde.

6. Verfahren nach Anspruch 5, wobei ein Abschnitt der cDNA-Fragmente von (iii) aus den 3'-Regionen der Ausgangs-Nukleinsäurepopulation stammt, und ein Abschnitt der cDNA-Fragmente von (iv) aus den 5'-Regionen der Ausgangs-Nukleinsäurepopulation stammt.

7. Verfahren nach Anspruch 6, wobei die Fragmente, die die 3'-Regionen oder die 5'-Regionen der Ausgangs-Nukleinsäurepopulation darstellen, im Anschluss an das Anbringen aus anderen cDNA-Fragmenten isoliert werden.

8. Verfahren nach Anspruch 2, wobei mindestens ein Verbindungsstück eine Bindungsstelle für einen Primer oder eine Polymerase umfasst, und wobei das Verfahren weiterhin den folgenden Schritt (c) und den vorhergehenden Schritt (d) umfasst:
Entfernen des unteren Strangs jedes Verbindungsstück-Erkennungssequenz-Konstrukts; und Neubilden des unteren Strangs durch Reverse Transkription, Primer-Verlängerung oder PCR-Amplifikation.

9. Verfahren nach Anspruch 1, wobei die erste Restriktionsendonuklease ausgewählt aus Bpml, Mmel, Gsul und Isoschizomeren davon.

10. Verfahren nach einem der Ansprüche 1 bis 9, wobei die Erkennungssequenzen mindestens zehn Basenpaare lang sind.

11. Verfahren nach Anspruch 2, weiterhin umfassend die Schritte:
(e) Anhängen einer Oligonukleotid-Markierung an jedes Erkennungssequenz-Fragment, so dass im Wesentlichen alle unterschiedlichen Erkennungssequenz-Fragmente an unterschiedlichen Oligonukleotid-Markierungen angebracht sind, um Markierungs-Erkennungssequenz-Konjugate zu bilden;
(f) Inkontaktbringen der Markierungs-Erkennungssequenz-Konjugate mit einer Bibliothek aus Markierungs-Komplementen jeweils auf einem getrennten Festphasen-Träger, und Hybridisieren der Markierungen mit ihren jeweiligen Komplementen, um klonale Subpopulationen von Erkennungssequenzen auf einem Festphasen-Träger zu bilden; und
(g) Sequenzieren einer Vielfalt von Erkennungssequenzen auf dem Festphasen-Träger.

12. Verfahren nach Anspruch 11, wobei das Anbringen der Markierungen umfasst:
(i) Verbinden der Erkennungssequenz-Fragmente mit einer Bibliothek aus Oligonukleotid-Markierungs-Vektoren, wobei jeder Markierungs-Vektor umfasst: eine linke Restriktionsspaltstelle, eine Oligonukleotid-Markierung, eine Klonierungsstelle zur Insertion des Erkennungssequenz-Fragments und eine rechte Restriktionsspaltstelle, um eine Vektorbibliothek aus Markierungs-Erkennungssequenz-Konjugaten zu bilden; und
(ii) Replizieren der Vektorbibliothek in einem Wirtsorganismus.

13. Verfahren nach Anspruch 12, wobei die Anzahl an unterschiedlichen Oligönukleotid-Markieruhgen in der Markierungs-Vektor-Bibliothek um einen Faktor von mindestens 100 größer ist als die Anzahl an unterschiedlichen Fragmenten, und wobei das Verfahren weiterhin den Schritt der Probeentnahme aus der Vektorbibliothek umfasst, so dass im Wesentlichen alle unterschiedlichen Polynukleotid-Fragmente innerhalb der Probe unterschiedlich angebrachte Oligonukleotid-Markierungen aufweisen.

14. Verfahren nach Anspruch 12, weiterhin umfassend:
(iii) Spalten der Markierungs-Erkennungssequenz-Konjugate aus der Vektorbibliothek;
(iv) Entfernen des unteren Strangs der Markierungskomponente der Markierungs-Erkennungssequenz-Konjugate;
(v) Inkontaktbringen der Markierungs-Erkennungssequenz-Konjugate mit einer Bibliothek aus Markierungskomplementen jeweils auf einem getrennten Festphasenträger, wodurch die Einzelstrang-Markierungen mit ihren jeweiligen Komplementen hybridisieren; und
(vi) Verbinden der unteren Stränge der Erkennungssequenz-Fragmente mit den Markierungs-Komplementen;
wodurch eine Bibliothek gebildet wird, die klonale Subpopulationen jeder Erkennungssequenz aus der Ausgangs-Polynukleotid-Population auf einem festen Träger umfasst.

## Revendications

1. Méthode de préparation d'un bibliothèque de séquences de signatures de la même longueur à partir d'une population d'acide nucléique source, la méthode consistant à :
(a) attacher à une extrémité de chacun d'une population de polynucléotides, un premier adaptateur contenant un site de reconnaissance pour une première endonucléase de restriction de façon que le site de scission pour l'endonucléase soit dans le polynucléotide, où la première endonucléase de restriction est une endonucléase de restriction du type IIs ayant un site de scission à au moins 16 nucléotides de son site de reconnaissance et où l'extrémité à laquelle l'adaptateur est attaché est la même pour chaque polynucléotide et est sélectionnée parmi : (i) l'extrémité 5' d'une transcription d'ADNc de pleine longueur, (ii) l'extrémité 3' d'une transcription d'ADNc à partir de laquelle la voie polyA/poly T a été éliminée, (iii) l'extrémité 5' d'un fragment d'ADNc produit par scission d'ADNc avec une endonucléase de restriction et (iv) l'extrémité 3' d'un fragment d'ADNc produit par scission d'ADNc avec une endonucléase de restriction ;
(b) scinder les polynucléotides avec la première endonucléase de restriction, pour produire une population de conjugués adaptateur-signature, chacun contenant une séquence de signature de la même longueur de l'acide nucléique source, au moins six paires de bases de long, ayant une extrémité nouvellement scindée ; et
(c) ligaturer aux extrémités nouvellement scindées desdites signatures dans lesdits conjugués adaptateur-signature, un second adaptateur, ledit second adaptateur contenant un ou plusieurs de (i) un site de liaison d'amorce et (ii) un site de reconnaissance et de scission pour une seconde endonucléase de restriction pour produire une bibliothèque de construction adaptateur-signature-adaptateur.

2. Méthode de la revendication 1, comprenant de plus l'étape de (d) faire digérer les constructions avec la seconde endonucléase et une endonucléase de restriction efficace pour scinder le premier adaptateur pour produire une bibliothèque de fragments de signature de la même longueur flanqués de sites de clonage.

3. Méthode de la revendication 1 ou 2, où l'étape d'attachement (a) est effectuée en phase en solution.

4. Méthode de l'une quelconque des revendications 1 à 3, où l'extrémité à laquelle le premier adaptateur est attaché est sélectionnée parmi : (i) l'extrémité 5' d'un ADNc de pleine longueur et (ii) l'extrémité 3' d'un ADNc de pleine longueur d'où la voie polyA a été enlevée.

5. Méthode de l'une quelconque des revendications 1 à 3, où l'extrémité à laquelle le premier adaptateur est attaché est sélectionnée parmi : (iii) l'extrémité 5' d'un fragment d'ADNc produit par scission d'ADNc avec une endonucléase de restriction, et (iv) l'extrémité 3' d'un fragment d'ADNc produit par scission d'ADNc avec une endonucléase de restriction.

6. Méthode de la revendication 5, où une portion desdits fragments d'ADNc de (iii) sont dérivés des régions 3' de ladite population d'acides nucléiques source et une portion desdits fragments d'ADNc de (iv) sont dérivés des régions 5' de ladite population d'acides nucléiques source.

7. Méthode de la revendication 6 où lesdits fragments représentant les régions 3' ou les régions 5' de ladite population d'acides nucléiques source sont isolés des autres fragments d'ADNc à la suite dudit attachement.

8. Méthode de la revendication 2, où au moins un adaptateur comprend un site de liaison pour une amorce ou une polymérase et la méthode comprend de plus à la suite de l'étape (c) et de l'étape précédente (d) :
l'enlèvement du brin inférieur de chaque construction adaptateur-signature ; et
la régénération du brin inférieur par transcription réverse, extension d'amorce ou amplification PCR.

9. Méthode de la revendication 1, où la première endonucléase de restriction est sélectionnée parmi BpmI, MmeI, Gsul et leurs isoschizomères.

10. Méthode de l'une quelconque des revendications 1 à 9, où lesdites signatures ont au moins dix paires de bases de long.

11. Méthode de la revendication 2 comprenant de plus les étapes de :
(e) attacher un marqueur oligonucléotidique à chaque fragment de signature de façon que sensiblement tous les fragments différents de signature des marqueurs oligonucléotidiques différents attachés pour former des conjugués marqueur-signature ;
(f) mettre en contact les conjugués marqueur-signature avec une bibliothèque de compléments de marqueur, chacun sur un support en phase solide séparé et hybrider les marqueurs à leurs compléments respectifs pour former des sous-populations clonales supportées en phase solide de séquences de signatures ; et
(g) séquencer un certain nombre de séquences de signatures supportées en phase solide.

12. Méthode de la revendication 11, où l'attachement desdits marqueurs consiste à :
(i) ligaturer les fragments de signature dans une bibliothèque de marqueurs oligonucléotidiques-vecteurs,
où chaque marqueur-vecteur comprend : un site de scission de restriction gauche, un marqueur oligonucléotidique, un site de clonage pour insertion du fragment de signature et un site de scission de restriction droit pour former une bibliothèque de vecteurs de conjugués marqueur-signature ; et
(ii) la réplication de ladite bibliothèque de vecteurs dans un organisme hôte.

13. Méthode de la revendication 12, où le nombre de marqueurs oligonucléotidiques différents dans la bibliothèque de marqueurs-vecteurs est plus grand que le nombre de fragments différents d'un facteur d'au moins 100, et comprenant de plus l'étape de prélever un échantillon de la bibliothèque de vecteurs de façon que sensiblement tous les fragments polynucléotidiques différents dans l'échantillon aient des marqueurs oligonucléotidiques différents attachés.

14. Méthode de la revendication 12, comprenant de plus :
(iii) la scission desdits conjugués marqueur-signature de la bibliothèque de vecteurs ;
(iv) l'élimination du brin inférieur du composant marqueur des conjugués marqueur-signature ;
(v) la mise en contact des conjugués marqueur-signature avec une bibliothèque de compléments de marqueur, chacun sur un support en phase solide séparé pour ainsi hybrider les marqueurs à un seul brin à leurs compléments respectifs ; et
(vi) la ligature des brins inférieurs des fragments de signature aux compléments de marqueurs ;
pour ainsi former une bibliothèque comprenant des sous-populations clonales supportées en phase solide de chaque séquence de signature provenant de la population de polynucléotides source.


### SEQUENCE LISTING

<110> Lynx Therapeutics, Inc.
<120> Constant Length Signatures for Parallel
   Sequencing of Polynucleotides
<130> 555258053W00
<140> Not Yet Assigned
   <141> Filed Herewith
<150> US 60/375,782
   <151> 2002-04-26
<160> 16
<170> FastSEQ for Windows Version 4.0
<210> 1
   <211> 73
   <212> DNA
   <213> Artificial Sequence
<220>
   <223> exemplary tag library
<221> misc_feature
   <222> (71) ... (73)
   <223> n = A,T,C or G
<400> 1
   agaattcggg ccttaattaa dddddddddd dddddddddd dddddddddd ddgggcccgc      60
   ataagtcttc nnn                                                         73
<210> 2
   <211> 18
   <212> DNA
   <213> Artificial Sequence
<220>
   <223> primer
<221> misc_feature
   <222> 7, 14, 15, 16, 17, 18
   <223> n = A,T,C or G
<400> 2
   atcactngga tccnnnnn 18
<210> 3
   <211> 20
   <212> DNA
   <213> Artificial Sequence
<220>
   <223> primer
<400> 3
   agaattcggg ccttaattaa 20
<210> 4
   <211> 22 -
   <212> DNA
   <213> Artificial Sequence
<220>
   <223> primer
<400> 4
   cgttcagagt tctacagtcc ga 22
<210> 5
   <211> 23
   <212> DNA
   <213> Artificial Sequence
<220>
   <223> adapter
<400> 5
   cgttcagagt tctacagtcc gac 23
<210> 6
   <211> 26
   <212> DNA
   <213> Artificial Sequence
<220>
   <223> adaptor bottom strand
<400> 6
   gatcgtcgga ctgtagaact ctgaac 26
<210> 7
   <211> 22
   <212> DNA
   <213> Artificial Sequence
<220>
   <223> primer
<400> 7
   agacttctac gcatctccga ca 22
<210> 8
   <211> 29
   <212> DNA
   <213> Artificial Sequence
<220>
   <223> adapter
<400> 8
   catgtcggag atgcgtagaa gtctgaacg 29
<210> 9
   <211> 22
   <212> DNA
   <213> Artificial Sequence
<220>
   <223> primer
<400> 9
   agccgtcagc atcattgagc at 22
<210> 10
   <211> 83
   <212> DNA
   <213> Artificial Sequence
<220>
   <223> adapter-signature-adapter construct
<221> misc_feature
   <222> (30) ... (46)
   <223> n = A,T,C or G
<400> 10
   cgttcagact tctacgcatc tccgacatgn nnnnnnnnnn nnnnnnggtt cagcaggaat 60
   gctcaatgat gctgacggct gtt 83
<210> 11
   <211> 23
   <212> DNA
   <213> Artificial Sequence
<220>
   <223> primer
<400> 11
   gctacacgat tctacagtct gga 23
<210> 12
   <211> 27
   <212> DNA
   <213> Artificial Sequence
<220>
   <223> adapter
<400> 12
   gatctccaga ctgtagaatc gtgtagc 27
<210> 13
   <211> 37
   <212> DNA
   <213> Artificial Sequence
<220>
   <223> adapter
<400> 13
   ggttcagcag gaatgctcaa tgatgctgac ggctgtt 37
<210> 14
   <211> 36
   <212> DNA
   <213> Artificial Sequence
<220>
   <223> adapter bottom strand
<221> misc feature
   <222> (35)...(36)
   <223> n = A,T,C or G
<400> 14
   agccgtcagc atcattgagc attcctgctg aaccnn 36
<210> 15
   <211> 28
   <212> DNA
   <213> Artificial Sequence
<220>
   <223> adapter dimer fragment
<221> misc feature
   <222> (14)...(15)
   <223> n = A,T,C or G
<400> 15
   ttcctgctga accnnggttc agcaggaa 28
<210> 16
   <211> 300
   <212> DNA
   <213> Artificial Sequence
<220>
   <223> cloning vector
<400> 16
   gaattctgaa taaatagcgc cagggttttc ccagtcacga cgtgtaaaac gacggccagt      60
   cgaccgtcca gacttctact acctcactta attaaggaat aggcctctcc tcgagctcgg     120
   taccgggccc gcttcacaga tgtcggctaa tgcataagtc ttcatctgca gaatcgatga     180
   ttgaagagcg atatcgctct tcaatcggat ccgatgctga caagatacga ccacgcggcc     240
   gcggtcatag ctgtttcctg ccacacaaca tacgagccgg aagctcaact aattaagctt     300